# EUROPÄISCHE PATENTSCHRIFT

(11) **EP 1 458 722 B1**
(45) Veröffentlichungstag und Bekanntmachung des Hinweises auf die Patenterteilung: **15.10.2008**
(21) Anmeldenummer: 02805744.6
(22) Anmeldetag: 08.11.2002
(51) Int. Cl.: C07D 487/04, A61K 31/505

(54) **PYRROLOPYRIMIDINE ALS PHOSPHODIESTERASE-VII-HEMMER**
PYRROLOPYRIMIDINES AS PHOSPHODIESTERASE VII INHIBITORS
PYRROLOPYRIMIDINES EN TANT D'AGENTS D'INHIBITION DE PHOSPHODIESTERASE-VII

(30) Priorität: 24.12.2001 DE 10163991
(43) Veröffentlichungstag der Anmeldung: 22.09.2004
(73) Patentinhaber: Merck Patent GmbH, 64293 Darmstadt (DE)
(72) Erfinder: EGGENWEILER, Hans-Michael, 64287 Darmstadt (DE); WOLF, Michael, 64287 Darmstadt (DE)
(86) Internationale Anmeldenummer: PCT/EP2002/012533
(87) Internationale Veröffentlichungsnummer: WO 2003/055882

(56) Entgegenhaltungen:
- EP-A- 0 005 205
- EP-A- 0 482 804
- WO-A-01/29049
- WO-A-01/32618
- WO-A-01/34601
- WO-A-01/57025
- WO-A-94/13676
- DE-A- 3 145 287
- K. EGER ET AL.: "Selected Reactions on the o-Aminonitrile System of Substituted Pyrroles" J. HETEROCYCL. CHEM., Bd. 24, Nr. 2, 1987, Seiten 425-430, XP002229256
- C. E. MÜLLER ET AL.: "7-Deaza-2-phenyladenines: Structure-Activity Relationships of Potent A1 Selective Receptor Antagonists" J. MED. CHEM., Bd. 33, Nr. 10, 1990, Seiten 2822-2828, XP002229257
- M. ADENOT ET AL.: "Interest of cluster significance analysis in structure-affinity relationships for non-xanthine heterocyclic antagonists of adenosine" EUR. J. MED. CHEM., Bd. 32, Nr. 6, 1997, Seiten 493-504, XP002229258
- A. MIYASHITA ET AL.: "Preparation of heteroarenecarbonitriles by reaction of haloheteroarenes with potassium cyanide catalyzed by sodium p-toluenesulfinate" HETEROCYCLES, Bd. 39, Nr. 1, 1994, Seiten 345-356, XP009004660
- G. FOLKERS, H.-D. HÖLTJE: "Active site molecular modelling of xanthine oxidase inhibitors with antiinflammatory activity" J. MOLECULAR GRAPHICS, Bd. 3, Nr. 4, 1985, Seiten 146-150, XP009004653
- D. STEINHILBER ET AL.: "New Class of 5-Lipoxygenase Inhibitors: Correlation Between Inhibition of LTB4 Production and Chemiluminescence of Human Polymorphonuclear Granulocytes" PHARM. RESEARCH, Bd. 3, Nr. 5, 1986, Seiten 271-277, XP009004659
- F. JOHANNSEN ET AL.: "Reactions of Heterocyclic o-Aminonitriles with Acetic Formic Anhydride" CHEMICA SCRIPTA, Bd. 26, Nr. 2, 1986, Seiten 347-351, XP009004655
- H. J. ROTH, K. EGER: "Synthese von Pyrrolo[2,3-d]pyrimidinen" ARCH. PHARMAZ., Bd. 308, Nr. 4, 1975, Seiten 252-258, XP002059633
- J. W. DALY ET AL.: "7-Deaza-9-phenyladenines. A new class of adenosine receptor antagonists" BIOCHEM. PHARMACOL., Bd. 37, Nr. 19, 1988, Seiten 3749-3753, XP002928089
- A. O. ABDELHAMID ET AL.: "Reactions with heterocyclic enaminonitriles: synthesis of pyrrolo[2,3-b]pyridine, pyrrolo[2,3-d]pyrimidine and pyrrole derivatives" HETEROCYCLES, Bd. 27, Nr. 8, 1988, Seiten 1861-1866, XP000973572
- S. KLUMPP ET AL.: "Pyrrolo[2,3-d]pyrimidines as inhibitors of cAMP Phosphodiesterase" BIOCHEM. PHARMACOL., Bd. 38, Nr. 6, 1989, Seiten 949-953, XP001117494
- A. MIYASHITA ET AL.: "Catalytic Action of Azolium Salts. IX. Synthesis of 6-Aroyl-9H-purines and Their Analogues by Nucleophilic Aroylation Catalyzed by Imidazolium or Benzimidazolium Salt" CHEM. PHARM. BULL., Bd. 46, Nr. 3, 1998, Seiten 390-399, XP001135025
- G. FOLKERS: "Rezeptoraffinität von Xanthinoxidase-Inhibitoren - strukturelle Voraussetzungen" DEUTSCHE APOTHEKER ZEITUNG, Bd. 126, Nr. 41, 1986, Seiten 2243-2247, XP001135024

## Beschreibung

Die Erfindung betrifft Verbindungen ausgewählt aus der Gruppe
5-Isopropyl-4-oxo-7-p-tolyl-4,7-dihydro-3*H*-pyrrolo[2,3-*d*]-pyrimidin-6-carbonsäureethylester,
5-Methyl-4-oxo-7-(3-chlorphenyl)-4,7-dihydro-3*H*-pyrrolo[2,3-*d*]-pyrimidin-6-carbonsäureethylester,
5-Methyl-4-oxo-7-(2-chlorphenyl)-4,7-dihydro-3*H*-pyrrolo[2,3-*d*]-pyrimidin-6-carbonsäureethylester,
5-Methyl-4-oxo-7-(2-fluorphenyl)-4,7-dihydro-3*H*-pyrrolo[2,3-*d*]-pyrimidin-6-carbonsäureethylester,
5-Propyl-4-oxo-7-(2-chlorphenyl)-4,7-dihydro-3*H*-pyrrolo[2,3-*d*]-pyrimidin-6-carbonsäureethylester,
5-Methyl-4-oxo-7-(4-chlorphenyl)-4,7-dihydro-3*H*-pyrrolo[2,3,-*d*]-pyrimidin-6-carbonsäureethylester,
5-Methyl-4-oxo-7-(2-chlorphenyl)-4,7-dihydro-3*H*-pyrrolo[2,3-*d*]-pyrimidin-6-carbonsäuremethylester,
5-Methyl-4-oxo-7-phenyl-4,7-dihydro-3*H*-pyrrolo[2,3-*d*]-pyrimidin-6-carbonsäuremethylester,
5-Methyl-4-oxo-7-(2-thienyl)-4,7-dihydro-3*H*-pyrrolo[2,3-*d*]-pyrimidin-6-carbonsäuremethylester,
sowie ihre pharmazeutisch verwendbaren Salze, Solvate und Stereoisomere, einschließlich deren Mischungen in allen Verhältnissen.

Pyrrolderivate als Phosphodiesterase VII-Hemmer sind z.B. aus der WO 01/32618 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die erfindungsgemäßen Verbindungen und ihre Salze und Solvate bei guter Verträglichkeit sehr wertvolle pharmakologische Eigenschaften besitzen.
Insbesondere zeigen sie eine spezifische Inhibierung der "Rolipram insensitiven" cAMP Phosphodiesterase (PDE VII).

Die biologische Aktivität der erfindungsgemäßen Verbindungen kann nach Methoden bestimmt werden, wie sie z.B von M.A. Giembycz et al. in Br. J. Pharmacol. (1996), 118, 1945-1958 beschrieben sind.
Die Affinität der Verbindungen für cAMP-Phosphodiesterase (PDE VII) wird durch die Ermittlung ihrer I₅₀-Werte (Konzentration des Inhibitors, die benötigt wird, um eine 50 %ige Inhibierung der Enzymaktivität zu erreichen) bestimmt.
Zur Durchführung der Bestimmungen wurden anstelle von T-Lymphozyten homogenisierte SK-N-SH Neuroblastomazellen verwendet, zur PDE III Inhibierung wurde CI-930 eingesetzt. Hierbei handelt es sich um einen selektiven PDE III Inhibitor (J.A. Bristol et al., J. Med. Chem. 1984, 27(9), 1099-1101).
Alternativ wird SK-N-SH durch HUT-78 ersetzt und statt CI-930 wird mit Trequensin inhibiert (D. Ruppert et al., Life Sci. 31:2037, 1982).

Die erfindungsgemäßen Verbindungen können zur Behandlung von asthmatischen Erkrankungen eingesetzt werden.
Die antiasthmatische Wirkung kann z. B. analog der Methode von T. Olsson, Acta allergologica 26, 438-447 (1971), bestimmt werden.

Da cAMP knochenabbauende Zellen hemmt und knochenaufbauende Zellen stimuliert (S. Kasugai et al., M 681 und K. Miyamoto, M 682, in Abstracts of the American Society for Bone and Mineral Research 18th Annual Meeting, 1996), können die Verbindungen der Formel I zur Behandlung von Osteoporose eingesetzt werden.

Die Verbindungen zeigen außerdem eine antagonistische Wirkung auf die Produktion von TNFα (Tumor Nekrose Faktor) und eignen sich daher zur Behandlung von allergischen und entzündlichen Krankheiten, Autoimmunkrankheiten, wie z.B. rheumatoide Arthritis, multiple Sklerose, Morbus Crohn, Diabetes mellitus oder ulzerative Kolitis, Transplantatabstoßungsreaktionen, Kachexie und Sepsis.
Die antiinflammatorische Wirkung der Substanzen und ihre Wirksamkeit zur Behandlung von z.B. Autoimmunerkrankungen wie multipler Sklerose oder rheumatoider Arthritis, kann analog den Methoden von N. Sommer et al., Nature Medicine 1, 244-248 (1995) oder L. Sekut et al., Clin. Exp. Immunol. 100, 126-132 (1995) bestimmt werden.
Die Verbindungen können zur Behandlung von Kachexie eingesetzt werden. Die anti-kachektische Wirkung kann in TNF-abhängigen Modellen der Kachexie geprüft werden (P. Costelli et al., J. Clin. Invest. 95, 2367ff. (1995); J.M. Argiles et al., Med. Res. Rev. 17, 477ff. (1997)). Die PDE VII-Inhibitoren können auch das Wachstum von Tumorzellen hemmen und sind deshalb für die Tumortherapie geeignet (für PDE IV-Hemmer vgl. D. Marko et al., Cell Biochem. Biophys. 28, 75ff. (1998)).

Sie können weiterhin für die Therapie von Sepsis und zur Behandlung von Gedächtnisstörungen, Atherosklerose, atopische Dermatitis und AIDS eingesetzt werden, ferner zur Behandlung T-Zellen-abhängiger Krankheiten (L.Li et al., Science, 1999, 283, 848-851).
Die Wirkung von z.B. PDE IV-Inhibitoren bei der Behandlung von Asthma, entzündlichen Erkrankungen, Diabetes mellitus, atopischer Dermatitis, Psoriasis, AIDS, Kachexie, Tumorwachstum oder Tumormetastasen ist z.B. in der EP 77 92 91 beschrieben.

Die Verbindungen können als Arzneimittelwirkstoffe in der Human- und Veterinärmedizin eingesetzt werden. Ferner können sie als Zwischenprodukte zur Herstellung weiterer Arzneimittelwirkstoffe eingesetzt werden.

Die Erfindung betrifft weiterhin die Verwendung von Typ-7-Phosphodiesteraseinhibitoren (PDE VII-Inhibitoren) zur Behandlung von Erkrankungen.

Es wird auf WO 01/57025 verwiesen, aus der spezielle Pyrimidinderivate als PDE IV-Inhibitoren, ihre Verwendung zur Behandlung von Erkrankungen sowie Kombinationen mit anderen Arzneistoffen bekannt sind.

Gegenstand der Erfindung ist dementsprechend insbesondere die Verwendung der erfindungsgemäßen Verbindungen und ihren physiologisch unbedenklichen Salzen und Solvaten zur Herstellung eines Arzneimittels zur Behandlung eines Patienten, der an einer Erkrankung oder einem Leiden leidet, die vom PDE VII-Isozym in seiner Rolle bei der Regulierung der Aktivierung und Degranulation humaner Eosinophiler vermittelt wird.
Gegenstand der Erfindung sind auch die erfindungsgemäßen Verbindungen und ihren physiologisch unbedenklichen Salzen und Solvaten zur Behandlung oder Prophylaxe von Krankheiten, die durch Verbindungen mit PDE VII-inhibitorischer Aktivität bekämpft oder beeinflußt werden können.

Aus WO 01/57025 sind verschiedene In-vitro-Assays und Tiermodellversuche bekannt, die genug Daten bereitstellen können, um den therapeutischen Nutzen der erfindungsgemäßen Verbindungen, analog den dort genannten PDE IV-Inhibitoren, zu definieren und nachzuweisen.

Die erfindungsgemäßen Verbindungen hemmen das PDE VII-Isozym und eignen sich daher aufgrund der wesentlichen Rolle, die die PDE-VII-Isozymfamilie in der Physiologie aller Säugetiere spielt, für verschiedenste therapeutische Anwendungen. Bei der enzymatischen
Rolle der PDE VII-Isozyme handelt es sich um die intrazelluläre Hydrolyse von Adenosin-3',5'-Monophosphat (cAMP) in Leukozyten vor Erreichen des Entzündungszustands. cAMP wiederum ist für die Vermittlung der Wirkung zahlreicher Hormone im Körper verantwortlich und die Hemmung von PDE VII spielt daher eine wesentliche Rolle bei verschiedenen physiologischen Vorgängen. Es liegt umfassende Fachliteratur vor, in der die Wirkungen von PDE-Inhibitoren auf verschiedene Entzündungsreaktionen der Zelle beschrieben werden, zu denen außer der Erhöhung von cAMP auch die Hemmung der Superoxidproduktion, Degranulation, Chemötaxis und Freisetzung des Tumor Nekrose Faktors (TNF) in Eosinophilen, Neutrophilen und Monozyten zählt.

Die erfindungsgemäßen Verbindungen können auch in Form ihrer Tautomeren vorliegen.

Die Erfindung betrifft außerdem die optisch aktiven Formen (Stereoisomere), die Enantiomere, die Racemate, die Diastereomere und die Hydrate und Solvate dieser Verbindungen. Unter Solvaten der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Monohydrate, Dihydrate oder Alkoholate.

Unter pharmazeutisch verwendbaren Derivaten werden z.B. die Salze der erfindungsgemäßen Verbindungen sowie sogenannte Prodrug-Verbindungen verstanden.
Unter Prodrug-Derivaten werden z.B. Verbindungen verstanden, die z.B. mit Alkyl- oder Acylgruppen, Zuckern oder Oligopeptiden modifiziert wurden und die im Organismus rasch gespalten werden und so die erfindungsgemäßen Wirkstoffe abgeben.

Dazu zählen auch biologisch abbaubare Polymerderivate der erfindungsgemäßen Verbindungen wie z.B. in Int. J. Pharm. 115, 61-67 (1995) beschrieben.

Die Verbindungen und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart), beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die erfindungsgemäßen Verbindungen werden vorzugsweise z.B. nach dem folgenden Reaktionsschema hergestellt: 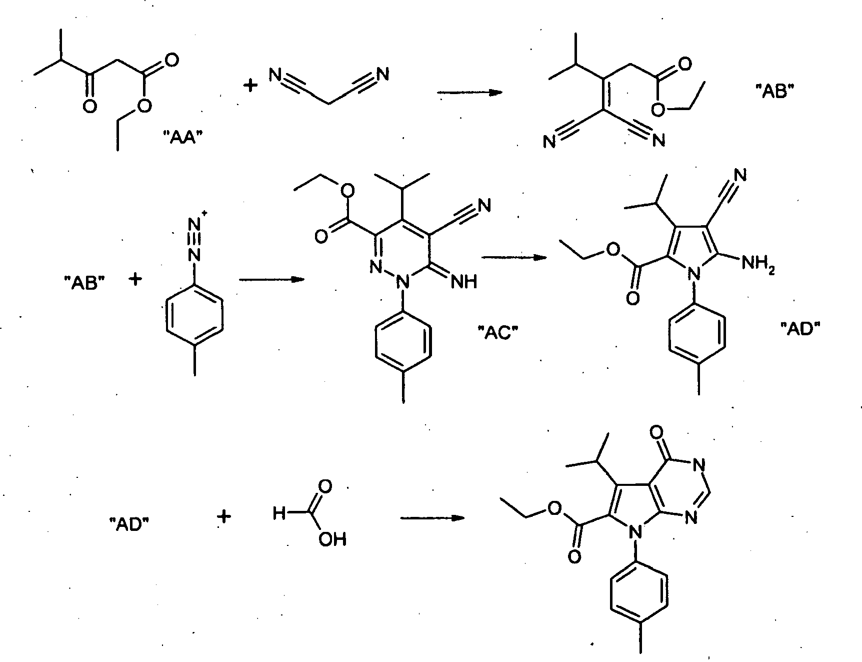 "AD" entspricht einer Verbindung der Formel II.
HCOOH entspricht einer Verbindung der Formel III.

Im einzelnen erfolgt die Umsetzung der Verbindungen der Formel II mit den Verbindungen der Formel III in Gegenwart oder Abwesenheit eines inerten Lösungsmittels bei Temperaturen zwischen etwa -20 und etwa 150°, vorzugsweise zwischen 80 und 120°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1 ,2-Dichlorethan, Tetrachlorkohlenstoff, Chloroform oder Dichlormethan; Alkohole wie Methanol; Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat, oder Gemische der genannten Lösungsmittel.

Die erfindungsgemäßen Verbindungen werden ferner z.B. analog der Cyclisierung von "AE" hergestellt, das einer Verbindung der Formel IV entspricht. 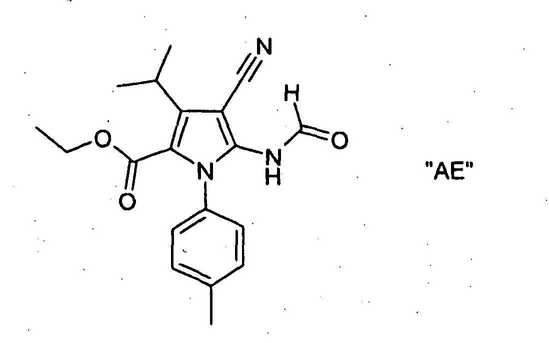 "AE" tritt als Zwischenprodukt bei der Reaktion von "AD" mit Ameisensäure auf. "AE" wird entweder in einer Eintopfreaktion weiterverarbeitet oder es wird isoliert und anschließend cyclisiert.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der erfindungsgemäßen Verbindungen werden größtenteils konventionell hergestellt. Sofern die Verbindung eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der erfindungsgemäßen Verbindungen zählen ebenfalls dazu. Bei bestimmten erfindungsgemäßen Verbindungen lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der erfindungsgemäßen Verbindungen die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der erfindungsgemäßen Verbindungen, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser- als auch öllösliche erfindungsgemäße Verbindungen hergestellt werden.

Zu den oben genannten pharmazeutischen Salzen, die bevorzugt sind, zählen Acetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Zu den pharmakokinetischen Eigenschaften des Wirkstoffs, die günstig beeinflußt werden können, zählen z.B. die Art, wie dieser Wirkstoff durch Zellmembranen hindurch transportiert wird, was wiederum die Absorption, Verteilung, biologische Umwandlung und Exkretion dieses Wirkstoffs direkt und positiv beeinflussen kann. Obwohl der Verabreichungsweg der pharmazeutischen Zusammensetzung wichtig ist und verschiedene anatomische, physiologische und pathologische Aspekte die biologische Verfügbarkeit entscheidend beeinflussen können, hängt die Löslichkeit des Wirkstoffs üblicherweise von der Art seiner jeweiligen Salzform, die verwendet wird, ab. Weiterhin ist dem Fachmann deutlich, daß eine wäßrige Lösung des Wirkstoffs für die rascheste Absorption des Wirkstoffs in den Körper eines behandelten Patienten sorgt, während Lipidlösungen und -Suspensionen sowie feste Dosierungsformen zu einer weniger raschen Absorption des Wirkstoffs führen.

Die orale Aufnahme eines Wirkstoffs stellt aus Sicherheits-, Bequemlichkeits- und Sparsamkeitsgründen den am stärksten bevorzugten Verabreichungsweg dar, die Absorption einer derartigen oralen Dosierungsform kann jedoch durch physikalische Eigenschaften wie Polarität, durch Reizung der Magen-Darm-Schleimhaut hervorgerufenes Erbrechen, Abbau durch Verdauungsenzyme und niedrigen pH, ungleichmäßige Absorption oder Propulsion in Gegenwart von Nahrungsmitteln oder anderen Arzneistoffen sowie Stoffwechsel durch Enzyme der Schleimhaut, der Darmflora oder der Leber gestört werden. Die Formulierung des Wirkstoffs als unterschiedliche pharmazeutisch unbedenkliche Salzformen kann zur Überwindung oder Verringerung eines oder mehrerer der oben genannten Probleme im Zusammenhang mit der Absorption oraler Dosierungsformen wirksam sein.

Eine gemäß den hier beschriebenen Verfahren hergestellte Verbindung läßt sich aus der Reaktionsmischung, in der sie endgültig hergestellt wird, auf jedem beliebigen üblichen Weg, mit dem der Chemiker auf dem Gebiet der organischen Synthese vertraut ist, abtrennen. Die abgetrennten Verbindungen lassen sich nach bekannten Verfahren reinigen. Für die Trennung und Reinigung lassen sich verschiedene Verfahren und Techniken verwenden, darunter z.B. Destillation, Umkristallisieren, Säulenchromatographie, Ionenaustauschchromatographie, Gelchromatographie, Affinitätschromatographie, präparative Dünnschichtchromatographie sowie Lösungsmittelextraktion.

### Stereoisomere

Eine Verbindung kann dergestalt sein, daß die Atome, aus denen sie besteht, trotz identischer Verknüpfungen räumlich auf zwei oder mehr Wege angeordnet sein können. Diese Verbindung liegt infolgedessen in Form von Stereoisomeren vor. Cis-trans-Isomerie ist nur eine Art der Stereoisomerie. Sind die Stereoisomere Bild und Spiegelbild, die nicht zur Deckung gebracht werden können, so handelt es sich um Enantiomere mit Chiralität oder Händigkeit, da ein oder mehrere asymmetrische Kohlenstoffatome in der sie bildenden Struktur vorliegen. Enantiomere sind optisch aktiv und daher unterscheidbar, da sie die Ebene von polarisiertem Licht gleich stark, jedoch in entgegengesetzte Richtungen drehen.

Liegen in einer Verbindung zwei oder mehr asymmetrische Kohlenstoffatome vor, so existieren an jedem dieser Kohlenstoffatome zwei mögliche Konfigurationen. Liegen zwei asymmetrische Kohlenstoffatome vor, existieren zum Beispiel vier mögliche Stereoisomere. Weiterhin lassen sich diese vier möglichen Stereoisomere in sechs mögliche Stereoisomerenpaare einteilen, die sich voneinander unterscheiden. Ein Molekülpaar mit mehr als einem asymmetrischen Kohlenstoff muß an jedem asymmetrischen Kohlenstoff unterschiedliche Konfigurationen aufweisen, um als Enantiomere zu gelten. Diejenigen Paare, die sich nicht wie Enantiomere verhalten, weisen eine unterschiedliche stereochemische Beziehung auf, die als diastereomere Beziehung bezeichnet wird. Stereoisomere, die keine Enantiomere sind, werden als Diastereoisomere oder häufiger Diastereomere bezeichnet.

Alle diese gut bekannten Aspekte der Stereochemie der Verbindungen werden als Teil der vorliegenden Erfindung betrachtet. Die vorliegende Erfindung umfaßt daher Verbindungen, die Stereoisomere sind, und, falls es sich bei diesen um Enantiomere handelt, die einzelnen Enantiomere, racemische Mischungen dieser Enantiomere sowie künstliche, d.h. synthetische Mischungen, die Anteile dieser Enantiomere enthalten, die sich von den in einer racemischen Mischung beobachteten Anteilen dieser Enantiomere unterscheiden. Umfaßt eine Verbindung Stereoisomere, bei denen es sich um Diastereomere handelt, so umfaßt diese Verbindung die einzelnen Diastereomere sowie Mischungen von zwei oder mehr beliebigen dieser Diastereomeren in beliebigen Anteilen.

Zur Erläuterung soll folgendes dienen: existiert ein einziges asymmetrisches Kohlenstoffatom in einer Verbindung, was zu ihren (-)(R)- und (+)(S)-Enantiomeren führt, so umfaßt diese Verbindung alle ihrer pharmazeutisch unbedenklichen Salzformen, Prodrugs und Metaboliten, die therapeutisch wirksam und nützlich zur Behandlung der bzw. Vorbeugung gegen die im weiteren Text beschriebenen Erkrankungen und Leiden sind. Liegt eine Verbindung der Formel I in Form von (-)(R)-und (+)(S)-Enantiomeren vor, so umfaßt diese Verbindung auch das (+)(S)-Enantiomer allein oder das (- )(R)-Enantiomer allein, wenn die therapeutische Wirksamkeit insgesamt, im wesentlichen oder hauptsächlich in nur einem dieser Enantiomere vorliegt bzw. wenn unerwünschte Nebenwirkungen in nur einem dieser Enantiomere vorliegen. Falls zwischen den biologischen Eigenschaften der beiden Enantiomere im wesentlichen kein Unterschied existiert, so umfaßt diese Verbindung weiterhin das (+)(S)-Enantiomer und das (-)(R)-Enantiomer gemeinsam als racemische Mischung oder nichtracemische Mischung in einem beliebigen Verhältnis von entsprechenden Anteilen.

Die spezifischen biologischen Wirkungen bzw. physikalischen und chemischen Eigenschaften eines Paares oder Satzes von Enantiomeren einer Verbindung - falls vorhanden - können zum Beispiel zur Bildung eines therapeutischen Endprodukts die Verwendung dieser Enantiomere in bestimmten Verhältnissen nahelegen. Zur Veranschaulichung soll folgendes dienen: falls ein Enantiomerenpaar existiert, können die Enantiomere in Verhältnissen wie 90% (R) - 10% (S), 80% (R) - 20% (S), 70% (R) - 30% (S), 60% (R) - 40% (S), 50% (R) - 50% (S), 40% (R) - 60% (S), 30% (R) - 70% (S), 20% (R) - 80% (S) und 10% (R) - 90% (S) verwendet werden. Nach der Auswertung der Eigenschaften der verschiedenen Enantiomeren einer Verbindung - falls solche existieren - läßt sich die entsprechende Menge eines oder mehrerer dieser Enantiomere mit bestimmten erwünschten Eigenschaften, die das therapeutische Endprodukt bilden, auf einfache Weise ermittelt werden.

### Isotope

Es ist weiterhin vorgesehen, daß eine Verbindung isotopenmarkierte Formen davon umfaßt. Eine isotopenmarkierte Form einer Verbindung ist mit dieser Verbindung bis auf die Tatsache, daß eines oder mehrere Atome der Verbindung durch ein Atom bzw. Atome mit einer Atommasse oder Massenzahl ersetzt wurden, die sich von der Atommasse oder Massenzahl des Atoms, das üblicherweise natürlich vorkommt, unterscheidet, identisch. Zu den Isotopen, die leicht im Handel erhältlich sind und in eine Verbindung nach gut bekannten Verfahren eingebaut werden können, zählen zum Beispiel Isotope von Wasserstoff, Kohlenstoff, Stickstoff, Sauerstoff, Phosphor, Fluor und Chlor, z.B. ²H, ³H, ¹³C, ¹⁴C, ¹⁵N, ¹⁸O, ¹⁷O, ³¹P, ³²P, ³⁵S, ¹⁸F bzw. ³⁶Cl. Eine Verbindung der Formel I, eines ihrer Prodrugs oder jeweils ein pharmazeutisch unbedenkliches Salz davon, die eines oder mehrere der oben genannten Isotope und/oder andere Isotope von anderen Atomen enthält, ist als Bestandteil der vorliegenden Erfindung vorgesehen. Eine isotopenmarkierte Verbindung läßt sich auf vielerlei nützliche Art verwenden. Zum Beispiel eignet sich eine isotopenmarkierte Verbindung, in die z.B. ein Radioisotop wie ³H oder ¹⁴C eingebaut worden ist, für Assays zur Verteilung des Arzneistoffs und/oder Substratgewebes. Diese Radioisotope, d.h. Tritium (³H) und Kohlenstoff-14 (¹⁴C), sind aufgrund ihrer einfachen Herstellung und ausgezeichneten Nachweisbarkeit besonders bevorzugt. Der Einbau schwererer Isotope, z.B. Deuterium (²H), in eine Verbindung weist therapeutische Vorteile aufgrund der höheren Stabilität dieser isotopenmarkierten Verbindung im Metabolismus auf. Höhere Stabilität in Metabolismus bedeutet unmittelbar eine erhöhte Halbwertszeit in vivo oder niedrigere Dosierungen, was unter den meisten Umständen eine bevorzugte Ausführungsform der vorliegenden Erfindung darstellen würde. Eine isotopenmarkierte Verbindung läßt sich üblicherweise durch Durchführung der in den Syntheseschemata und der damit in Zusammenhang stehenden Beschreibung, im Beispielteil und im Herstellungsteil im vorliegenden Text offenbarten Vorgehensweisen herstellen, wobei ein nicht isotopenmarkierter Reaktionspartner durch einen leicht verfügbaren isotopenmarkierten Reaktionspartner ersetzt wird.

Zur Manipulation des oxidativen Metabolismus der Verbindung über den primären kinetischen Isotopeneffekt kann auch Deuterium (²H) in eine Verbindung eingebaut werden. Beim primären kinetischen Isotopeneffekt handelt es sich um eine Veränderung der Geschwindigkeit einer chemischen Reaktion aufgrund des Austausches isotopischer Kerne, was wiederum durch die Änderung der für die Bildung kovalenter Bindungen im Anschluß an diesen isotopischen Austausch erforderlichen Grundzustandsenergien verursacht wird. Der Austausch eines schwereren Isotops führt üblicherweise zu einer Erniedrigung der Grundzustandsenergie für eine chemische Bindung und verursacht so eine Verringerung der Geschwindigkeit bei einem geschwindigkeitslimitierenden Bindungsbruch. Findet der Bindungsbruch an bzw. in der Nähe einer Sattelpunktregion entlang der Koordinate einer Reaktion mit mehreren Produkten statt, so können sich die Produktverteilungsverhältnisse stark ändern. Zur Erläuterung: Wird Deuterium an ein Kohlenstoffatom in einer nichtaustauschbaren Position gebunden, so sind Geschwindigkeitsunterschiede von k_{M}/k_{D} = 2-7 typisch. Wird dieser Geschwindigkeitsunterschied erfolgreich auf eine oxidationsanfällige Verbindung angewandt, so kann sich dadurch das Profil dieser Verbindung in vivo drastisch ändern und zu verbesserten pharmakokinetischen Eigenschaften führen.

Bei der Entdeckung und Entwicklung von Therapeutika versucht der Fachmann, pharmakokinetische Parameter zu optimieren und gleichzeitig wünschenswerte In-vitro-Eigenschaften beizubehalten. Man kann vernünftig annehmen, daß viele Verbindungen mit schlechten pharmakokinetischen Profilen gegenüber dem oxidativen Metabolismus anfällig sind. Aus derzeitig verfügbaren In-vitro-Assays mit Lebermikrosomen erhält man wertvolle Informationen über den Verlauf dieses oxidativen Metabolismus, aufgrund dessen wiederum deuterierte Verbindungen mit einer verbesserten Stabilität durch Resistenz gegenüber einem derartigen oxidativen Metabolismus rational gestaltet werden können. So gelangt man zu wesentlichen Verbesserungen der pharmakokinetischen Profile der Verbindungen, die sich quantitativ als erhöhte In-vivo-Halbwertszeit (T/2), Konzentration bei maximaler therapeutischer Wirkung (Cₘₐₓ), Fläche unter der Dosis-Wirkungskurve (AUC) sowie F und als verringerte Clearance, Dosis und Materialkosten ausdrücken lassen.

Zur Veranschaulichung des Obigen soll folgendes dienen: eine Verbindung mit mehrfachen potentiellen Angriffsstellen für den oxidativen Metabolismus, z.B. Wasserstoffatome an einem Benzylrest und Wasserstoffatome, die an ein Stickstoffatom gebunden sind, wird als Reihe von Analogen hergestellt, in denen verschiedene Kombinationen von Wasserstoffatomen durch Deuteriumatome ersetzt werden, so daß einige, die meisten oder alle dieser Wasserstoffatome durch Deuteriumatome ersetzt sind. Durch Bestimmungen der Halbwertszeit gelangt man zu einer günstigen und genauen Bestimmung, wie sehr sich die Verbesserung der Widerstandsfähigkeit gegenüber oxidativen Metabolismen verbessert hat. Auf diese Weise wird bestimmt, daß aufgrund eines derartigen Austausches von Wasserstoff gegen Deuterium die Halbwertszeit der Ausgangsverbindung um bis zu 100% verlängert werden kann.

Der Austausch von Wasserstoff gegen Deuterium in einer Verbindung läßt sich auch dazu verwenden, um zu einer günstigen Änderung des Stoffwechselproduktspektrums der Ausgangsverbindung zwecks Verringerung oder Ausschluß von unerwünschten toxischen Stoffwechselprodukten zu gelangen. Entsteht zum Beispiel ein toxisches Stoffwechselprodukt aufgrund der Spaltung einer oxidativen KohlenstoffWasserstoff (C-H)-Bindung kann vernünftigerweise angenommen werden, daß das deuterierte Analog die Produktion des unerwünschten Stoffwechselprodukts wesentlich verringert oder ausschließt, sogar dann, wenn es sich bei der jeweiligen Oxidation nicht um einen geschwindigkeitsbestimmenden Schritt handelt. Weitere Informationen zum Stand der Technik in bezug auf den Austausch von Wasserstoff gegen Deuterium finden sich z.B. bei Hanzlik et al., J. Org. Chem. 55, 3992-3997, 1990, Reider et al., J. Org. Chem. 52, 3326-3334, 1987, Foster, Adv. Drug Res. 14, 1-40, 1985, Gillette et al., Biochemistry 33(10), 2927-2937, 1994, und Jarman et al., Carcinogenesis 16(4), 683-688, 1993.

### Therapeutische Anwendungen

Die Erfindung betrifft weiterhin die Verwendung von Verbindungen zur Behandlung von Myocarderkrankungen.

Koronare Herzerkrankungen stellen die häufigste Todesursache in der westlichen Welt dar. Bei einem kritisch verengten Herzkranzgefäß kann ein reduzierter Blutstrom zu Myokardischämie führen. Wird mit einer Reperfusion begonnen, so führt dies je nach der Schwere des vorangegangenen Ischämieschubs zu einer reversiblen oder nichtreversiblen Schädigung des Myokards, die durch langandauernde Depression oder den irreversiblen Verlust der Kontraktionsfähigkeit gekennzeichnet ist. Je nach der Größe der betroffenen Myokardregion kann es zu einem akuten oder chronischen Herzversagen kommen.

Ein besonderes klinisches Problem bei dem oben beschriebenen Fall ist die Entstehung einer Restenose nach anfänglich erfolgreicher Reperfusion durch PTKA, auch nach Implantation eines Stents, nach Thrombolyse oder nach Transplantation eines aorto-koronaren Bypasses. Aus experimentellen Tierversuchen wie klinischen Versuchen gibt es Hinweise, daß bei den verschiedenen oben genannten Herzerkrankungen, d.h. koronare Herzerkrankung selbst, reversible oder irreversible Myokardischämie/Reperfusionsschädigung, akutes oder chronisches Herzversagen und Restenose, darunter auch In-Stent-Restenose und Stent-in-Stent-Restenose, Entzündungsvorgänge eine zufällige Rolle spielen. An diesen Entzündungsvorgängen sind existierende sowie einwandernde Makrophagen sowie Neutrophile und TH₁- und TH₂-Helferzellen beteiligt. Diese Leukozytenreaktion führt zu dem charakteristischen Cytokinmuster, an dem TNF-α, IL-1β, IL-2 und IL-6 sowie IL-10 und IL-13 beteiligt sind (Pulkki KJ: Cytokines and cardiomyocyte death. Ann. Med. 1997 29: 339-343.
Birks EJ, Yacoub MH: The role of nitric oxide and cytokines in heart failure. Coron.Artery.Dis. 1997 8: 389-402).
Die Bildung dieser Spezies wurde an menschlichen Patienten mit Myokardischämie nachgewiesen. Im Tiermodell zeigt sich, daß die Cytokinproduktion der Einwanderung peripherer Makrophagen und Neutrophilen korreliert ist, wodurch die Schädigung in das noch intakte Myokard verschleppt werden kann.

Der wichtigste Faktor bei der Cytokinantwort ist jedoch TNF-α, der entzündliche und pro-apoptotische Reaktionen vereinigt und außerdem eine direkte negativ-ionotrope Wirkung auf die Herzmyozyten ausübt. (Ceconi C, Curello S, Bachetti T, Corti A, Ferrari R: Tumor necrosis factor in congestive heart failure: a mechanism of disease for the new millennium? Prog.Cardiovasc.Dis. 1998 41: 25-30.

Mann DL: The effect of tumor necrosis factor-alpha on cardiac structure and function: a tale of two cytokines. J.Card.Fail. 1996 2: S165-S172. Squadrito F, Altavilla D, Zingarelli B, et al.: Tumor necrosis factor involvement in myocardial ischaemia-reperfusion injury. Eur.J.Pharmacol. 1993 237: 223-230).

An Tiermodellen des Myokardinfarkts wurde gezeigt, daß TNF-α während der Reperfusionsphase rasch freigesetzt wird (Herskowitz A, Choi S, Ansari AA, Wesselingh S: Cytokine mRNA expression in postischemic/reperfused myocardium. Am.J.Pathol. 1995 146: 419-428) und daß die Schutzwirkungen von Arzneistoffen wie Dexamethason (Arras M; Strasser R, Mohri M, et al.: Tumor necrosis factor-alpha is expressed by monocytes/macrophages following cardiac microembolization and is antagonized by cyclosporine. Basic.Res.Cardiol. 1998 93: 97-107), Cyclosporin A (Arras M, Strasser R, Mohri M, et al.: Tumor necrosis factor-alpha is expressed by monocytes/macrophages following cardiac microembolization and is antagonized by cyclosporine. Basic.Res.Cardiol. 1998 93: 97-107.
Squadrito F, Altavilla D, Squadrito G, et al.: Cyclosporin-A reduces leukocyte accumulation and protects against myocardial ischaemia reperfusion injury in rats. Eur.J.Pharmacol. 1999 364: 159-168) oder Clorichromen (Squadrito F, Altavilla D, Zingarelli B, et al.: The effect of cloricromene, a coumarine derivate, on leukocyte accumulation, myocardial necrosis and TNF-alpha production in myocardial ischaemiareperfusion injury. Life Sci. 1993 53: 341-355) mit einer Verringerung von zirkulierendem TNF- α einhergehen.

PDE VII-Inhibitoren sind wirksame Antagonisten der Makrophagen- und T-Zellen-Cytokinproduktion. Sie hemmen außerdem die Proliferation von T-Zellen. Die Hemmung von PDE VII kann daher eine günstige Wirkung bei denjenigen Myokarderkrankungen, bei denen ein kausaler Zusammenhang mit der Cytokinproduktion und mit entzündlichen Vorgängen besteht, ausüben.

Das Ziel der Erfindung bestand darin, neue Verwendungsmöglichkeiten von Verbindungen mit wertvollen Eigenschaften zu entdecken, insbesondere von denjenigen, die zur Herstellung von Arzneimitteln verwendet werden können.

Es wurde gefunden, daß die erfindungsgemäßen Verbindungen und ihre Salze äußerst wertvolle pharmakologische Eigenschaften mit guter Verträglichkeit zur Behandlung von Myokarderkrankungen in sich vereinigen.

Die Erfindung sieht vorzugsweise die Verwendung der erfindungsgemäßen Verbindungen zur Herstellung eines Arzneimittels zur Behandlung von Myokarderkrankungen vor, wobei diese Myokarderkrankungen entzündliche und immunologische Merkmale aufweisen.

Am stärksten bevorzugt sieht die Erfindung die Verwendung der erfindungsgemäßen Verbindungen zur Herstellung eines Arzneimittels zur Behandlung von koronaren Herzerkrankungen, reversibler oder irreversibler Myokardischämie/Reperfusionsschädigung, akutem oder chronischem Herzversagen und Restenose, darunter auch In-Stent-Restenose und Stent-in-Stent-Restenose, vor.

Bevorzugt sieht die Erfindung die Verwendung der erfindungsgemäßen Verbindungen I zur Herstellung eines Arzneimittels zur Behandlung oder Vorbeugung von einer oder mehreren aus der Gruppe der folgenden Erkrankungen, krankhaften Störungen und Leiden vor:
Asthma jeglicher Art, Ätiologie oder Pathogenese, oder Asthma aus der Gruppe atopisches Asthma, nichtatopisches Asthma, allergisches Asthma, IgE-vermitteltes atopisches Asthma, Bronchialasthma, essentielles Asthma, Primärasthma, durch pathophysiologische Störungen hervorgerufenes endogenes Asthma, durch Umweltfaktoren hervorgerufenes exogenes Asthma, essentielles Asthma unbekannter oder inapparenter Ursache, nichtatopisches Asthma, bronchitisches Asthma, emphysematöses Asthma, durch Belastung induziertes Asthma, Berufsasthma, durch Bakterien-, Pilz-, Protozoen- oder Virusinfektion hervorgerufenes infektallergisches Asthma, nichtallergisches Asthma, inzipientes Asthma, "wheezy infant syndrome";
chronische oder akute Bronchokonstriktion, chronische Bronchitis, Obstruktion der kleinen Atemwege sowie Emphysem;
obstruktive oder entzündliche Atemwegserkrankung jeglicher Art, Ätiologie oder Pathogenese, oder eine obstruktive oder entzündliche Atemwegserkrankung aus der Gruppe Asthma; Staublunge, chronische eosinophile Pneumonie; chronischer obstruktive pulmonaler Krankheit (COPD); COPD inklusive chronische Bronchitis, Lungenemphysem oder damit assoziierte Atemnot, durch irreversible, fortschreitende Obstruktion der Atemwege gekennzeichnete COPD, Schocklunge (adult respiratory distress syndrome, ARDS) sowie Verschärfung der Überempfindlichkeit der Atemwege aufgrund Therapie mit anderen Arzneistoffen;
Staublunge jeglicher Art, Ätiologie oder Pathogenese, oder Staublunge aus der Gruppe Aluminose oder Aluminiumstaublunge, Anthrakose(-Asthma), Asbestose oder Asbeststaublunge, Chalikose oder Kalkstaublunge, durch Einatmen von Straußenfedernstaub verursachte Ptilose, durch Einatmung von Eisenteilchen verursachte Siderose, Silikose oder Steinstaublunge, Byssinose oder Baumwollstaubpneumokoniose sowie Talkpneumokoniose;
Bronchitis jeglicher Art, Ätiologie oder Pathogenese, oder Bronchitis aus der Gruppe akute Bronchitis, akute laryngotracheale Bronchitis, durch Erdnüsse ausgelöste Bronchitis, Bronchialkatarrh, kruppöse Bronchitis, Bronchitis ohne Auswurf, infektiöse Asthmabronchitis, Bronchitis mit Auswurf, Staphylokokken- oder Streptokokkenbronchitis; sowie Vesikulärbronchitis;
Bronchiektasie jeglicher Art, Ätiologie oder Pathogenese, oder Bronchiektasie aus der Gruppe zylindrische Bronchiektasie, sackförmige Bronchiektasie, spindelförmige Bronchiektasie, Bronchiolendilatation, zystische Bronchiektasie, Bronchiektasie ohne Auswurf, sowie follikuläre Bronchiektasie;
jahreszeitlich bedingte allergische Rhinitis, perenniale allergische Rhinitis, oder Sinusitis jeglicher Art, Ätiologie oder Pathogenese, oder Sinusitis aus der Gruppe eitriger oder nichteitriger Sinusitis, akute oder chronische Sinusitis, Ethmoiditis, Stirnhöhlenentzündung, Kieferhöhlenentzündung oder Sphenoiditis;
rheumatoide Arthritis jeglicher Art, Ätiologie oder Pathogenese, oder rheumatoide Arthritis aus der Gruppe akute Arthritis, akute Gichtarthritis, primär-chronische Polyarthritis, Osteoarthrose, Infektarthritis, Lyme-Arthritis, progrediente Arthritis, Arthritis psoriatica, sowie Spondylarthritis;
Gicht sowie mit Entzündung assoziierte Fieber bzw. mit Entzündung assoziierte Schmerz;
eine mit Eosinophilen in Zusammenhang stehende krankhafte Störung jeglicher Art, Ätiologie oder Pathogenese, oder eine mit Eosinophilen in Zusammenhang stehende krankhafte Störung aus der Gruppe Eosinophilie, eosinophiles Lungeninfiltrat, Löffler-Syndrom, chronische eosinophile Pneumonie, tropische Lungeneosinophilie, bronchopneumonische Aspergillose, Aspergillom, eosinophiles Granulom, allergische granulomatöse Angiitis bzw. Churg-Strauss-Syndrom, Polyarteriitis nodosa (PAN), sowie systemische Vasculitis necroticans;
atopische Dermatitis, allergische Dermatitis, oder allergisches oder atopisches Ekzem;
Nesselsucht jeglicher Art, Ätiologie oder Pathogenese, oder Nesselsucht aus der Gruppe immunbedingte Nesselsucht, Komplement-bedingte Nesselsucht, durch Nesselsucht auslösendes Material induzierte Nesselsucht, durch physikalische Reize ausgelöste Nesselsucht, durch Streß ausgelöste Nesselsucht, idiopatische Nesselsucht, akute Nesselsucht, chronische Nesselsucht, angioneurotisches Ödem, Urticaria cholinergica, Kälteurtikaria in ihrer autosomal-dominanten Form oder in ihrer erworbenen Form, Kontakturtikaria, Urticaria gigantean sowie Papelurtikaria;
Konjunktivitis jeglicher Art, Ätiologie oder Pathogenese, oder Konjunktivitis aus der Gruppe Conjunctivitis actinica, akute katarrhalische Konjunktivitis, akute contagiöse Konjunktivitis, allergische Konjunktivitis, atopische Konjunktivitis, chronische katarrhalische Konjunktivitis, eitrige Konjunktivitis sowie Frühjahrskonjunktivitis;
Uveitis jeglicher Art, Ätiologie oder Pathogenese oder Uveitis aus der Gruppe Entzündung der ganzen Uvea oder eines Teils davon, Uveitis anterior, Iritis, Cyclitis, Iridocyclitis, granulomatöse Uveitis, nichtgranulomatöse Uveitis, phakoantigene Uveitis, Uveitis posterior, Choroiditis sowie Choriorethinitis;
Schuppenflechte;
multiple Sklerose jeglicher Art, Ätiologie oder Pathogenese, oder multiple Sklerose aus der Gruppe primär progrediente multiple Sklerose sowie multiple Sklerose mit schubweisem Verlauf und Neigung zu Remissionen;
Autoimmun-/Entzündungserkrankungen jeglicher Art, Ätiologie oder Pathogenese, oder eine Autoimmun-/Entzündungserkrankung aus der Gruppe autoimmunhämatologische Störungen, hämolytische Anämie, aplastische Anämie, aregenerative Anämie, idiopatische thrombozytopene Purpura, systemischer Lupus erythematosus, Polychondritis, Skleroderm, Wegener-Granulomatose, Lichtkrankheit, chronisch-aktive Hepatitis, Myasthenia gravis, Stevens-Johnson-Syndrom, idiopathische Sprue, Autoimmun-Reizkolonerkrankungen, Colitis ulcerosa, Morbus Crohn, endokrine Opthamopathy, Basedow-Krankheit, Sarkoidose, Alveolitis, chronische Hypersensitivitätspneumonitis, primär biliäre Zirrhose, Insulinmangeldiabetes oder Typ 1 Diabetes mellitus, Uveitis anterior, granulomatöse Uveitis oder Uveitis posterior, Keratoconjunctivitis sicca, Keratoconjunctivitis epidemica, (diffuse) interstitielle Lungenfibrose, Lungenzirrhose, Mukoviszidose, Arthritis psoriatica, Glomerulonephritis mit und ohne Nephrose, akute Glomerulonephritis, idiopathische Nephrose, Minimal-Change-Nephropathie, entzündliche/ hyperproliferative Hauterkrankungen, Schuppenflechte, atopische Dermatitis, Kontaktdermatitis, allergische Kontaktdermatitis, familiärer gutartiger Pemphigus, Pemphigus erythematosus, Pemphigus foliaceus sowie Pemphigus vulgaris;
Vorbeugung einer Fremdtransplantatabstoßung nach Organtransplantation,
Reizdarm (inflammatory bowel disease, IBD) jeglicher Art, Ätiologie oder Pathogenese, oder Reizdarm aus der Gruppe ulzerative Kolitis (UC), kollagenöse Kolitis, Colitis polyposa, transmurale Kolitis sowie Morbus Crohn (CD);
septischer Schock jeglicher Art, Ätiologie oder Pathogenese, oder septischer Schock aus der Gruppe Nierenversagen, akutes Nierenversagen, Kachexie, Malariakachexie, hypophysäre Kachexie, uremämische Kachexie, Herzkachexie, Cachexia suprarenalis bzw. Addison-Krankheit, karzinomatöse Kachexie sowie Kachexie auf Grund von Infektion durch Human Immunodeficiency Virus (HIV);
Leberschädigung;
pulmonaler Hochdruck sowie durch Sauerstoffmangel hervorgerufener pulmonaler Hochdruck;
Knochenschwunderkrankungen, primäre Osteoporose und sekundäre Osteoporose;
krankhafte Störungen des Zentralnervensystems jeglicher Art, Ätiologie oder Pathogenese, oder eine krankhafte Störung des Zentralnervensystems aus der Gruppe Depression, Morbus Parkinson, Lern- und Gedächtnisstörungen, tardive Dyskinesie, Drogenabhängigkeit, arteriosklerotische Demenz, sowie Demenz als Begleiterscheinung von Chorea Huntington, Morbus Wilson, Paralysis agitans sowie Thalamusatrophien;
Infektionen, insbesondere Virusinfektionen, wobei diese Viren die Produktion von TNF-α in ihrem Wirt erhöhen oder wobei diese Viren gegenüber Hinaufregulierung von TNF-α in ihrem Wirt empfindlich sind, so daß ihre Replikation oder andere wichtigen Aktivitäten behindert werden, darunter Viren aus der Gruppe HIV-1, HIV-2 und HIV-3, Zytomegalievirus, CMV; Grippe, Adenoviren und Herpesviren, darunter Herpes zoster und Herpes simplex;
Hefe- und Pilzinfektionen, wobei diese Hefen und Pilze gegenüber Hinaufregulierung durch TNF-α empfindlich sind oder die TNF-α-Produktion in ihrem Wirt auslösen, z.B. Pilzmeningitis, insbesondere bei gemeinsamer Verabreichung mit anderen Arzneistoffen der Wahl zur Behandlung systemischer Hefe- und Pilzinfektionen, darunter den Polymycinen, z.B. Polymycin B, Imidazolen, z.B. Clotrimazol, Econazol, Miconazol und Ketoconazol, den Triazolen, z.B. Fluconazol und Itranazol, sowie den Amphotericinen, z.B. Amphotericin B und liposomales Amphotericin B, was jedoch keine Einschränkung darstellen soll.
Ischämie-Reperfusionsschädigung, Autoimmundiabetes, retinale Autoimmunität, chronische lymphozytische Leukämie, HIV-Infektionen, Lupus erythematosus, Nieren- und Harnleitererkrankungen, krankhafte Urogenital- und Gastrointestinalstörungen, sowie Prostataerkrankungen.

Insbesondere eignen sich die erfindungsgemäßen Verbindungen zur Behandlung von (1) Entzündungserkrankungen und -leiden inklusive Gelenkentzündung, rheumatoide Arthritis, rheumatoide Spondylitis, Osteoarthritis, Reizdarm, ulzerative Kolitis, chronische Glomerulonephritis, Dermatitis sowie Morbus Crohn, (2) Erkrankungen und Leiden der Atemwege, inklusive Asthma, Schocklunge, chronische Pulmonitis, Bronchitits, chronische obstruktive Atemwegerkrankung sowie Silikose, (3) Infektionskrankheiten und -leiden inklusive Sepsis, septischer Schock, endotoxischer Schock, gramnegative Sepsis, toxisches Schocksyndrom, durch Bakterien-, Virus- oder Pilzinfektionen hervorgerufenes Fieber bzw. Myalgie, sowie Grippe; (4) Immunerkrankungen und -leiden inklusive Autoimmundiabetes, systemischer Lupus erythematosis, GvH-Reaktion, Abstoßung von Fremdtransplantaten,multiple Sklerose, Schuppenflechte und allergische Rhinitis, sowie (5) weitere Erkrankungen und Leiden inklusive Knochenresorptionserkrankungen, Reperfusionsschädigung, sekundäre Kachexie aufgrund Infektion oder Malignität, sekundäre Kachexie aufgrund AIDS, Infektion mit Human Immune Deficiency Virus (HIV), oder AIDS-related-Complex (ARC), Keloidbildung, Narbengewebsbildung, Typ 1 Diabetes mellitus sowie Leukämie.

### Pharmazeutische Zusammensetzungen und Formulierungen

Die folgende Beschreibung betrifft die Art und Weise, auf die die erfindungsgemäßen Verbindungen, gewünschtenfalls zusammen mit anderen Therapeutika oder Nichttherpeutika, mit überwiegend üblichen pharmazeutisch unbedenklichen Trägern kombiniert werden, wodurch man zu Dosierungsformen gelangt, die sich für die unterschiedlichen Verabreichungswege, die für einen gegebenen Patienten verwendet werden, und die Erkrankung, krankhafte Störung oder das Leiden, für die bzw. das ein gegebener Patient behandelt wird, eignen.

Die erfindungsgemäßen pharmazeutischen Zusammensetzungen umfassen eine oder mehrere beliebige der oben beschriebenen erfindungsgemäßen Inhibitorverbindungen oder eines ihrer pharmazeutisch unbedenklichen Salze wie ebenfalls oben beschrieben zusammen mit einem pharmazeutisch unbedenklichen Träger gemäß den Eigenschaften und dem erwarteten Verhalten solcher Träger, die dem Fachmann gut bekannt sind.

Die Menge an Wirkstoff, die mit den Trägermaterialien kombiniert werden kann, um so eine einzelne Dosierungsform zu bilden, hängt von dem behandelten Patienten und dem jeweiligen Verabreichungsweg ab. Es ist jedoch klar, daß ein bestimmtes Dosierungs- und Behandlungsschema für einen bestimmten Patienten von verschiedensten Faktoren, darunter der Wirksamkeit der jeweils verwendeten Verbindung, dem Alter, Körpergewicht, allgemeinem Gesundheitszustand, Geschlecht, der Ernährung, dem Verabreichungszeitpunkt, der Ausscheidungsgeschwindigkeit, der Arzneistoffkombination und dem Ermessen des behandelnden Arztes sowie der Schwere der jeweils behandelten Krankheit abhängt. Die Wirkstoffmenge kann auch von dem Therapeutikum oder Prophylaktikum, das gegebenenfalls mit dem Wirkstoff gemeinsam verabreicht wird, abhängen.

Die erfindungsgemäßen Verbindungen lassen sich in der Form von Säuren, Estern oder anderen chemischen Verbindungsklassen, zu denen die beschriebenen Verbindungen zählen, verwenden. Die vorliegende Erfindung umfaßt auch die Verwendung dieser Verbindungen in der Form von pharmazeutisch unbedenklichen Salzen, die sich von verschiedenen organischen und anorganischen Säuren und Basen ableiten. Ein Wirkstoff mit einer bevorzugten Verbindung wird häufig in der Form eines ihrer Salze verwendet, insbesondere wenn diese Salzform dem Wirkstoff im Vergleich zu der freien Form des Wirkstoffs oder einer zuvor verwendeten anderen Salzform des Wirkstoffs verbesserte pharmakokinetische Eigenschaften verleiht. Die pharmazeutisch unbedenkliche Salzform des Wirkstoffs kann auch diesem Wirkstoff erst eine erwünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Zu den pharmakokinetischen Eigenschaften des Wirkstoffs, die günstig beeinflußt werden können, zählen z.B. die Art, wie dieser Wirkstoff durch Zellmembranen hindurch transportiert wird, was wiederum die Absorption, Verteilung, biologische Umwandlung und Exkretion dieses Wirkstoffs direkt und positiv beeinflussen kann. Obwohl der Verabreichungsweg der pharmazeutischen Zusammensetzung wichtig ist und verschiedene anatomische, physiologische und pathologische Aspekte die biologische Verfügbarkeit entscheidend beeinflussen können, hängt die Löslichkeit des Wirkstoffs üblicherweise von der Art seiner jeweiligen Salzform, die verwendet wird, ab. Weiterhin ist dem Fachmann deutlich, daß eine wäßrige Lösung des Wirkstoffs für die rascheste Absorption des Wirkstoffs in den Körper eines behandelten Patienten sorgt, während Lipidlösungen und -suspensionen sowie feste Dosierungsformen zu einer weniger raschen Absorption des Wirkstoffs führen. Die orale Aufnahme eines Wirkstoffs stellt aus Sicherheits-, Bequemlichkeits- und Sparsamkeitsgründen den am stärksten bevorzugten Verabreichungsweg dar, die Absorption einer derartigen oralen Dosierungsform kann jedoch durch physikalische Eigenschaften wie Polarität, durch Reizung der Magen-Darm-Schleimhaut hervorgerufenes Erbrechen, Abbau durch Verdauungsenzyme und niedrigen pH, ungleichmäßige Absorption oder Propulsion in Gegenwart von Nahrungsmitteln oder anderen Arzneistoffen sowie Stoffwechsel durch Enzyme der Schleimhaut, der Darmflora oder der Leber gestört werden. Die Formulierung des Wirkstoffs als unterschiedliche pharmazeutisch unbedenkliche Salzformen kann zur Überwindung oder Verringerung eines oder mehrerer der oben genannten Probleme im Zusammenhang mit der Absorption oraler Dosierungsformen wirksam sein.

Die erfindungsgemäßen pharmazeutischen Zusammensetzungen umfassen eine oder mehrere der oben beschriebenen Inhibitorverbindungen oder eines ihrer ebenfalls wie oben beschriebenen pharmazeutisch unbedenklichen Salze zusammen mit einem pharmazeutisch unbedenklichen Träger entsprechend den Eigenschaften und dem erwarteten Verhalten solcher dem Fachmann gut bekannten Träger.

Der Begriff "Träger" umfaßt im vorliegenden Zusammenhang unbedenkliche Streckmittel, Exzipientien, Hilfsstoffe, Konstituentien, Lösungsvermittler, viskositätsmodifizierende Mittel, Konservierungsmittel und andere Mittel, die dem Fachmann gut bekannt sind, um der endgültigen pharmazeutischen Zusammensetzung günstige Eigenschaften zu verleihen. Zur Veranschaulichung dieser Träger folgt nun eine kurze Übersicht pharmazeutisch unbedenklicher Träger, die sich bei den erfindungsgemäßen pharmazeutischen Zusammensetzungen verwenden lassen, und im Anschluß daran eine genauere Beschreibung der unterschiedlichen Arten von Bestandteilen. Zu typischen Trägern zählen die folgenden, was jedoch keinesfalls eine Einschränkung darstellen soll: Ionenaustauscherzusammensetzungen, Aluminiumoxid, Aluminiumstearat, Lezitin, Serumproteine, z.B. Humanserumalbumin, Phosphate, Glycin, Sorbinsäure, Kaliumsorbat, Partialglyceridmischungen gesättigter Pflanzenfettsäuren, hydrierte Palmöle, Wasser, Salze oder Elektrolyte, z.B. Prolaminsulfat, Dinatriumhydrogenphosphat, Kaliumhydrogenphosphat, Natriumchlorid und Zinksalze, kolloidale Silica, Magnesiumtrisilicat, Polyvinylpyrrolidon, Substanzen auf Zellulosegrundlage, z.B. Natriumcarboxymethylcellulose, Polyethylenglycol, Polyacrylate, Wachse, Polyethylen-Polyoxypropylen-Blockpolymere sowie Wollfett.

Insbesondere umfaßt die bei den erfindungsgemäßen pharmazeutischen Zusammensetzungen verwendeten Träger unterschiedliche Klassen und Arten von Zusatzstoffen, die unabhängig aus den im wesentlichen in den folgenden Absätzen genannten Gruppen ausgewählt werden.

Ansäuernde und alkalisierende Mittel werden zugesetzt, um zu einem gewünschten oder vorbestimmten pH zu gelangen; sie umfassen Säuerungsmittel, z.B. Essigsäure, Eisessig, Apfelsäure und Propionsäure. Stärkere Säuren wie Salzsäure, Salpetersäure und Schwefelsäure können verwendet werden, sind jedoch weniger bevorzugt. Zu den alkalisierenden Mitteln zählen zum Beispiel Edetol, Kaliumcarbonat, Kaliumhydroxid, Natriumborat, Natriumcarbonat und Natriumhydroxid. Alkalisierende Mittel, die aktive Aminogruppen enthalten, wie Diethanolamin und Trolamin, können ebenfalls verwendet werden.

Soll die pharmazeutische Zusammensetzung als Aerosol unter beträchtlichem Druck abgegeben werden, so sind Aerosoltreibmittel erforderlich. Zu diesen Treibmitteln zählen zum Beispiel unbedenkliche Fluorchlorkohlenwasserstoffe wie Dichlordifluormethan, Dichlortetrafluorethan und Trichlormonofluormethan, Stickstoff, ein flüchtiger Kohlenwasserstoff wie Butan, Propan oder Isobutan, oder deren Mischungen.

Antimikrobielle Mittel, darunter Mittel gegen Bakterien, Pilze und Protozoen, werden zugegeben, wenn die pharmazeutische Zusammensetzung topisch auf Hautflächen aufgetragen wird, die wahrscheinlich einer schädigenden Umgebung ausgesetzt waren, oder Abschürfungen oder Schnitte erlitten haben, die die Haut für eine Infektion durch Bakterien, Pilzen oder Protozoen anfällig macht. Zu den antimikrobiellen Mitteln zählen Verbindungen wie Benzylalkohol, Chlorbutanol, Phenylethylalkohol, Phenylquecksilberacetat, Kaliumsorbat und Sorbinsäure. Zu den antifungalen Mitteln zählen Verbindungen wie Benzoesäure, Butylparaben, Ethylparaben, Methylparaben, Propylparaben und Natriumbenzoat.

Antimikrobielle Konservierungsstoffe werden den erfindungsgemäßen pharmazeutischen Zusammensetzungen zugegeben, um diese gegen das Wachstum von möglicherweise schädlichen Mikroorganismen zu schützen, die üblicherweise in die wäßrige Phase einwandern, in manchen Fällen jedoch auch in der Ölphase einer Zusammensetzung wachsen können. Es sind daher Konservierungsstoffe erwünscht, die sowohl in wäßrigen Medien als auch in Lipiden löslich sind. Zu geeigneten antimikrobiellen Konservierungsstoffen zählen z.B. p-Hydroxybenzoesäurealkylester, Propionatsalze, Phenoxyethanol, Methylparaben-Natrium, Propylparaben-Natrium, Natriumdehydroacetat, Benzalkoniumchlorid, Benzethoniumchlorid, Benzylalkohol, Hydantoinderivate, quartäre Ammoniumverbindungen und kationische Polymere, Imidazolidinylharnstoff, Diazolidinylharnstoff und Trinatriumethylendiamintetraacetat (EDTA).
Konservierungsstoffe werden vorzugsweise in Mengen von ungefähr 0,01 Gew.-% bis ungefähr 2,0 Gew.-% der Gesamtzusammensetzung eingesetzt.

Antioxidantien werden zugesetzt, um alle Bestandteile der pharma- , zeutischen Zusammensetzung gegen Schädigung oder Abbau durch Oxidationsmittel, die in der Zusammensetzung selbst oder in der Umgebung, in der sie verwendet werden, vorliegen, zu schützen, z.B. Anoxomer, Ascorbylpalmitat, Butylhydroxyanisol, Butylhydroxytoluol, hypophosphorige Säure, Kaliummetabisulfit, Gallensäurepropyl-, -octyl- und -dodecylester, Natriummetabisulfit, Schwefeldioxid und Tocopherole.

Puffersubstanzen werden verwendet, um einen erwünschten pH-Wert einer Zusammensetzung nach der Einstellung gegenüber den Auswirkungen externer Einflüsse und Gleichgewichtsverschiebungen von Bestandteilen der Zusammensetzungen aufrechtzuerhalten. Die Puffersubstanz kann unter den dem Fachmann auf dem Gebiet der Galenik bekannten ausgewählt werden, z.B. Calciumacetat, Kaliummetaphosphat, Kaliumdihydrogenphosphat und Weinsäure.

Chelatbildende Mittel dienen zur Erhaltung der Ionenstärke der pharmazeutischen Zusammensetzung; sie binden an schädigende Verbindungen und Metalle und entfernen diese dadurch wirksam. Dazu zählen z.B. Dikaliumedetat, Dinatriumedetat und EDTA.

Dermatologische Wirkstoffe werden den erfindungsgemäßen pharmazeutischen Zusammensetzungen dort zugegeben, wo diese topisch anzuwenden sind; dazu zählen z.B. Wundheilungsmittel wie Peptidderivate, Hefe, Panthenol, Hexylresorcin, Phenol, Tetracyclinhydrochlorid, Lamin und Kinetin; Retinoide zur Behandlung von Hautkrebs, z.B. Retinol, Tretinoin, Isotretinoin, Etretinat, Acitretin und Arotinoid, milde antibakterielle Mittel zur Behandlung von Hautinfektionen, z.B. Resorcin, Salicylsäure, Benzoylperoxid, Erythromycin-Benzoylperoxid, Erythromycin und Clindamycin; antifungale Mittel zur Behandlung von Tinea corporis, Tinea pedis, Candida-Infektionen und Tinea versicolor, z.B. Griseofulvin, Azole wie Miconazol, Econazol, Itraconazol, Fluconazol und Ketoconazol, sowie Allylamine wie Naftifin und Terfinafin; antivirale Mittel zur Behandlung von Herpes simplex der Haut, Gürtelrose und Windpocken, z.B. Acyclovir, Famciclovir und Valacyclovir, Antihistamine zur Behandlung von Juckreiz, atopischer Dermatitis und Kontaktdermatitis, z.B. Diphenhydramin, Terfenadin, Asternizol, Loratadin, Cetirizin, Acrivastin und Temelastin, Lokalanästhetika zur Linderung von Schmerzen, Reizung und Jucken, z.B. Benzocain, Lidocain, Dibucain und Pramoxinhydrochlorid, Lokalanalgetika zur Linderung von Schmerzen und Entzündungen, z.B. Salicylsäuremethylester, Kampfer, Menthol und Resorcin, topische Antiseptika zur Verhinderung von Infektionen, z.B. Benzalkoniumchlorid und Povidon-lod, sowie Vitamine und ihre Derivate, wie Tocopherol, Tocopherolacetat, Retinsäure und Retinol.

Dispergier- und Suspendiermittel werden als Hilfsstoffe bei der Herstellung stabiler Formulierungen eingesetzt, dazu zählen z.B. Poligeenan, Povidon und Siliziumdioxid.

Emollientia sind vorzugsweise nichtölige, wasserlösliche Stoffe, die die Haut erweichen und beruhigen, insbesondere Haut, die durch übermäßigen Wasserverlust trocken geworden ist. Solche Stoffe werden bei erfindungsgemäßen pharmazeutischen Zusammensetzungen verwendet, die zur topischen Anwendung bestimmt sind; dazu zählen z.B. Kohlenwasserstofföle und -wachse, Triglyceridester, acetylierte Monoglyceride, Methylester und andere Alkylester von C₁₀-C₂₀-Fettsäuren, C₁₀-C₂₀-Fettsäuren, C₁₀-C₂₀-Fettalkohole, Lanolin und Derivate, Ester von mehrwertigen Alkoholen, wie Polyethylenglykol (200-600), Polyoxyethylensorbitanfettsäureester, Wachsester, Phospholipide und Sterole; Emulgatoren zur Herstellung von Öl-in-Wasser-Emulsionen; Exzipientien, z.B. Laurocapram und Polyethylenglykolmonomethylether, Feuchthaltemittel, z.B. Sorbit, Glycerin und Hyaluronsäure, Salbengrundlagen, z.B. Vaselin, Polyethylenglykol, Lanolin und Poloxamer, Penetrationsförderer, z.B. Dimethylisosorbid, Diethylglykolmonoethylether, 1-Dodecylazacycloheptan-2-on und Dimethylsulfoxid (DMSO), Konservierungsstoffe, z.B. Benzalkoniumchlorid, Benzethoniumchlorid, p-Hydroxybenzoesäurealkylester, Hydantoinderivate, Cetylpyridiniumchlorid, Propylparaben, Quartärammoniumverbindungen, wie Kaliumbenzoat sowie Thimerosal; Sequestriermittel, darunter Cyclodextrine, Lösungsmittel, z.B. Aceton, Alkohol, Amylenhydrat, Butylalkohol, Maiskeimöl, Baumwollsamenöl, Essigester, Glycerin, Hexylenglykol, Isopropylalkohol, Isostearylalkohol, Methylalkohol, Methylenchlorid, Mineralöl, Erdnußöl, Phosphorsäure, Polyethylenglykol, Polyoxypropylen-15-stearylether, Propylenglykol, Propylenglykoldiacetat, Sesamöl sowie gereinigtes Wasser, Stabilisatoren, z.B. Calciumsaccharat und Thymol, Tenside, z.B. Lapyriumchlorid; Laureth-4, d.h. α-Dodecyl-ω-hydroxy-poly(oxy-1,2-ethandiyl)- oder Polyethylenglykolmonododecylether.

Emulgatoren, darunter emulgierende und verdickende Mittel und Emulsionshilfsstoffe, werden zur Herstellung von Öl-in-Wasser-Emulsionen verwendet, wenn diese die Grundlage der erfindungsgemäßen pharmazeutischen Zusammensetzungen bilden. Zu diesen Emulgatoren zählen z.B. nichtionogene Emulgatoren, wie C₁₀-C₂₀-Fettalkohole und die Kondensationsprodukte dieser Fettalkohole mit 2 bis 20 Mol Ethylenoxid oder Propylenoxid, das Kondensationsprodukt von (C₆-C₁₂)Alkylphenolen und 2 bis 20 Mol Ethylenoxid, Ethylenglykolmono- und -di-C₁₀-C₂₀-Fettsäureester, C₁₀-C₂₀-Fettsäuremonoglycerid, Diethylenglykol, Polyethylenglykole mit einem MG von 200-6000, Polypropylenglykole mit einem MG von 200-3000 und insbesondere Sorbit, Sorbitan, Polyoxyethylensorbit, Polyoxyethylensorbitan, hydrophile Wachsester, Cetostearylalkohol, Oleylalkohol, Lanolinalkohole, Cholesterin, Mono- und Diglyceride, Glycerylmonostearat, Polyethylenglykolmonostearat, Ethylenglykol- und Ppolyoxyethylenglykolmono- und -distearinsäuremischester, Propylenglykolmonostearat, sowie Hydroxypropylcellulose. Emulgatoren mit aktiven Aminogruppen können ebenfalls verwendet werden; dazu zählen typischerweise anionenaktive Emulgatoren wie Fettsäureseifen, z.B. Natrium-, Kalium- und Triethanolaminseifen der C₁₀-C₂₀-Fettsäuren, Alkalimetall-, Ammonium- oder substituierte Ammoniumsalze von (C₁₀-C₃₀)Alkylsulfat, (C₁₀-C₃₀)Alkylsulfonate und (C₁₀-C₅₀)alkylethoxyethersulfonate. Zu weiteren geeigneten Emulgatoren zählen Rizinusöl und hydriertes Rizinusöl, Lecithin; sowie Polymere der 2-Propensäure zusammen mit Acrylsäurepolymeren, die beide mit Saccharose- und/oder Pentaerythrit-Allylethern vernetzt sind und unterschiedliche Viskositäten aufweisen, diese sind durch die Produktbezeichnungen Carbomer 910, 934, 934P, 940, 941 und 1342 gekennzeichnet. Kationenaktive Emulgatoren mit aktiven Aminogruppen können ebenfalls verwendet werden, darunter diejenigen auf Grundlage der quarteren Ammonium-, Morpholinium- und Pyridiniumverbindungen. Ähnlich können amphotere Emulgatoren mit aktiven Aminogruppen wie Cocobetaine, Lauryldimethylaminoxid und Cocoylimidazolin verwendet werden. Zu den Emulgatoren und Verdickungsmitteln, die verwendet werden können, zählen auch Cetylalkohol und Natriumstearat sowie Emulsionshilfstoffe wie Ölsäure, Stearinsäure und Stearylalkohol.

Zu den Exzipientien zählen z.B. Laurocapram und Polyethylenglykolmonomethylether.
Soll die erfindungsgemäße pharmazeutische Zusammensetzung topisch angewndet werden, so können Penetrationsförderer verwendet werden, darunter z.B. Dimethylisosorbid, Diethylglykolmonoethylether, 1-Dodecylazacycloheptan-2-on und Dimethylsulfoxid (DMSO). Solche Zusammensetzungen enthalten typischerweise auch Salbengrundlagen, z.B. Vaselin, Polyethylenglykol, Lanolin und Poloxamer, bei dem es sich um ein Polyoxyethylen-Polyoxypropylen-Blockcopolymer handelt, das auch als Tensid oder Emulgator dienen kann.

Konservierungsstoffe werden verwendet, um erfindungsgemäße pharmazeutische Zusammensetzungen gegen Abbau durch Mikroorganismen der Umgebung zu schützen, dazu zählen z.B. Benzalkoniumchlorid, Benzethoniumchlorid, p-Hydroxybenzoesäurealkylester, Hydantoinderivate, Cetylpyridiniumchlorid, Monothioglycerin, Phenol, Phenoxyethanol, Methylparaben, Imidazolidinylharnstoff, Natriumdehydroacetat, Propylparaben, quartäre Ammoniumverbindungen, insbesondere Polymere wie Polixetoniumchlorid, Kaliumbenzoat, Natriumformaldehydsulfoxylat, Natriumpropionat sowie Thimerosal.

Sequestriermittel werden verwendet, um die Stabilität der erfindungsgemäßen pharmazeutischen Zusammensetzung zu verbessern; dazu zählen z.B. die Cyclodextrine, bei denen es um eine Familie natürlicher cyclischer Oligosaccharide handelt, die mit unterschiedlichen Stoffen Einschlußkomplexe bilden können und unterschiedliche Ringgrößen aufweisen, wobei man diejenigen mit 6, 7 und 8 Glucoseresten pro Ring üblicherweise als α-Cyclodextrine, β-Cyclodextrine bzw. γ-Cyclodextrine bezeichnet. Zu geeigneten Cyclodextrinen zählen z.B. α-Cyclodextrin, β-Cyclodextrin, γ-Cyclodextrin, δ-Cyclodextrin sowie kationisierte Cyclodextrine.

Zu den Lösungsmitteln, die bei der Herstellung der erfindungsgemäßen pharmazeutischen Zusammensetzungen verwendet werden können, zählen z.B. Aceton, Alkohol, Amylenhydrat, Butylalkohol, Maiskeimöl, Baumwollsamenöl, Essigester, Glycerin, Hexylenglykol, Isopropylalkohol, Isostearylalkohol, Methylalkohol, Methylenchlorid, Mineralöl, Erdnußöl, Phosphorsäure, Polyethylenglykol, Polyoxypropylen-15-stearylether, Propylenglykol, Propylenglykoldiacetat, Sesamöl und gereinigtes Wasser.

Zu den Stabilisatoren, die sich geeigneterweise verwenden lassen, zählen z.B. Calciumsaccharat und Thymol.
Verdickungsmittel werden typischerweise bei Formulierungen zur topischen Anwendung verwendet, um diesen die gewünschte Viskosität bzw. die gewünschten Handhabungseigenschaften zu verleihen; dazu zählen z.B. Cethylesterwachs, Myristylalkohol, Paraffin, synthetisches Paraffin, Emulgierwachs, mikrokristallines Wachs, gebleichtes Wachs und gelbes Wachs.

Zucker werden häufig verwendet, um den erfindungsgemäßen pharmazeutischen Zusammensetzungen verschiedene erwünschte Eigenschaften zu verleihen und um die erzielten Ergebnisse zu verbessern; dazu zählen z.B. Monosaccharide, Disaccharide und Polysaccharide wie Glucose, Xylose, Fruktose, Reose, Ribose, Pentose, Arabinose, Allose, Tallose, Altrose, Mannose, Galaktose, Laktose, Saccharose, Erythrose, Glyceraldehyd oder deren Kombinationen.

Tenside werden verwendet, um erfindungsgemäßen pharmazeutischen Zusammensetzungen mit mehreren Bestandteilen Stabilität zu verleihen, bereits vorhandene Eigenschaften dieser Zusammensetzungen zu verstärken und den Zusammensetzungen neue erwünschte Eigenschaften zu verleihen. Tenside werden als Netzmittel, Antischaummittel, zur Verringerung der Oberflächenspannung von Wasser sowie als Emulgatoren, Dispergatoren und Penetrationsförderer verwendet; dazu zählen z.B. Lapyriumchlorid; Laureth 4, d.h. α-Dodecyl-ω-hydroxy-poly(oxy-1,2-ethandiyl)- oder Polyethylenglykolmonododecylether, Laureth-9, d.h. ein Gemisch aus Polyethylenglykolmonododecylethern mit einem Durchschnitt von 9 Ethylenoxidgruppen pro Molekül, Monoethanolamin; Nonoxynol-4,-9 und -10, d.h. Polyethylenglykolmono(p-nonylphenyl)ether; Nonoxynol-15, d.h. α-(p-Nonylphenyl)-ω-hydroxypenta-deca(oxyethylen); Nonoxynol 30, d.h. α-(p-Nonylphenyl)-ω-hydroxytriaconta(oxyethylen), Poloxalene; d.h. nichtionogenes Polymer des Typs Polyethylenpolypropylenglykol, MG = ca. 3000, Poloxamer, das oben den Salbengrundlagen diskutiert wurde, Polyoxyl(8)-, -(40)- und -(50)-Stearat, d.h. Poly(oxy-1,2-ethanediyl)-α-hydro-ω-hydroxy-octadecanoat; Polyoxyl-10-oleylether, d.h. Poly(oxy-1,2-ethandiyl)-, α-[(Z)-9-Octadecenyl-ω-hydroxy-[Lakune], Polysorbat 20, d.h. Poly(oxy-1,2-ethandiyl)sorbitanmonododecanoat, Polysorbat 40, d.h. Poly(oxy-1,2-ethandiyl)sorbitanmonohexadecanoat, Polysorbat 60, d.h. Poly(oxy-1,2-ethandiyl)sorbitanmonooctadecanoat, Polysorbat 65, d.h. Poly(oxy-1,2-ethandiyl)sorbitantrioctadecanoat, Polysorbat 80, d.h. Poly(oxy-1,2-ethandiyl)sorbitanmono-9-octadecenoat, Polysorbat 85, d.h. Poly(oxy-1,2-ethandiyl)sorbitantri-9-octadecenoat, Natriumlaurylsulfat; Sorbitanmonolaurat, Sorbitanmonooleat, Sorbitanmonopalmitat, Sorbitanmonostearat, Sorbitansesquioleat, Sorbitantrioleat und Sorbitantristearat.

Die Herstellung der erfindungsgemäßen pharmazeutischen Zusammensetzungen erfolgt auf äußerst einfache Art, wie dies dem Durchschnittsfachmann gut bekannt ist. Handelt es sich bei den erfindungsgemäßen pharmazeutischen Zusammensetzungen um einfache wäßrige Lösungen bzw. Lösungen in anderen Lösungsmitteln, so werden die verschiedenen Bestandteile der Gesamtzusammensetzung in einer beliebigen praktischen Reihenfolge zusammengegeben, die hauptsächlich von Gründen der Bequemlichkeit bestimmt wird. Diejenigen Bestandteile, die eine geringere Löslichkeit in Wasser, jedoch eine ausreichende Löslichkeit in dem gleichen Hilfslösungsmittel mit Wasser aufweisen, lassen sich alle in diesem Hilfslösungsmittel auflösen, wonach der Wasseranteil des Trägers mit der Hilfslösung versetzt wird, wodurch sich die darin gelösten Stoffe im Wasser lösen. Zur Unterstützung dieses Dispergiervorgangs bzw. Lösungsvorgangs kann ein Tensid eingesetzt werden.

Sollen die erfindungsgemäßen pharmazeutischen Zusammensetzungen in Form von Emulsionen vorliegen, so werden die Bestandteile der pharmazeutischen Zusammensetzung gemäß den folgenden allgemeinen Vorgehensweisen zusammengegeben. Die geschlossene Wasserphase wird erst auf eine Temperatur im Bereich von ungefähr 60°C bis ungefähr 95°C, vorzugsweise ungefähr 70°C bis ungefähr 95°C, erhitzt, wobei die Wahl der verwendeten Temperatur von den physikalischen und chemischen Eigenschaften der Bestandteile, die die Öl-in-Wasser-Emulsion bilden, abhängt. Sobald die geschlossene Wasserphase die gewählte Temperatur erreicht hat, werden die Bestandteile der endgültigen Zusammensetzung, die in diesem Stadium zuzugeben sind, unter starkem Rühren mit dem Wasser vermischt und darin dispergiert. Als nächstes wird die Temperatur des Wassers auf ungefähr das Ausgangsniveau gebracht, wonach die Bestandteile der Zusammensetzung, die den nächsten Schritt bilden, zu der Zusammensetzungsmischung unter mäßigem Rühren zugegeben werden, und es wird ungefähr 5 bis ungefähr 60 Minuten, vorzugsweise ungefähr 10 bis ungefähr 30 Minuten, je nach den Bestandteilen der ersten zwei Stufen, weiter gemischt. Hiernach wird die Zusammensetzungsmischung passiv oder aktiv auf ungefähr 20°C bis ungefähr 55°C gekühlt, so daß in den verbleibenden Stufen weitere Komponenten zugegeben werden können; wonach so viel Wasser zugegeben wird, daß die ursprünglich bestimmte Konzentration in der Gesamtzusammensetzung erzielt wird.

Erfindungsgemäß können die pharmazeutischen Zusammensetzungen in Form eines sterilen Injektionspräparats, zum Beispiel einer sterilen wäßrigen oder öligen Suspension zur Injektion vorliegen. Diese Suspension läßt sich nach fachbekannten Techniken mit geeigneten Dispergier-, Netz- und Suspendiermitteln formulieren. Bei dem sterilen Injektionspräparat kann es sich auch um eine sterile Lösung oder Suspension zur Injektion in einem nichttoxischen parenteral unbedenklichen Verdünnungsmittel oder Lösungsmittel handeln, zum Beispiel in Form einer Lösung in 1,3-Butandiol. Zu den unbedenklichen Konstituentien und Lösungsmitteln, die verwendet werden können, zählen Wasser, Ringersche Lösung sowie isotonische Kochsalzlösung. Außerdem werden sterile stabilisierte Öle üblicherweise als Lösungs- oder Suspendiermittel verwendet. Jedes milde stabilisierte Öl, darunter auch synthetische Mono- oder Diglyceride, kann für diesen Zweck verwendet werden. Fettsäuren wie Ölsäure und seine Glyceridderivate eignen sich zur Herstellung von Iniectabilia, ebenso natürliche pharmazeutisch unbedenkliche Öle, wie Olivenöl oder Rizinusöl, insbesondere in Form ihrer Polyethoxylate. Diese Öllösungen oder -suspensionen können auch als Verdünnungs- oder Dispergiermittel einen langkettigen Alkohol wie RH, HCIX oder einen ähnlichen Alkohol enthalten.

Die erfindungsgemäßen pharmazeutischen Zusammensetzungen lassen sich oral in einer beliebigen oral unbedenklichen Dosierungsform verabreichen, darunter Kapseln, Tabletten, wäßrige Suspensionen oder Lösungen, was jedoch keine Einschränkung darstellen soll. Bei den Oraltabletten zählen Laktose und Maisstärke zu häufig verwendeten Trägern. Typischerweise werden auch Gleitmittel wie Magnesiumstearat zugegeben. Bei der oralen Verabreichung in Kapselform zählen Laktose und getrocknete Maisstärke zu nützlichen Streckmitteln. Sollen wäßrige Lösungen oral verwendet werden, so wird der Wirkstoff mit Emulgatoren und Suspendiermitteln vereinigt. Falls gewünscht können auch bestimmte Süßstoffe, Geschmacksstoffe oder Farbstoffe zugegeben werden. Die erfindungsgemäßen pharmazeutischen Zusammensetzungen können jedoch auch in Form von Suppositorien zur rektalen Verabreichung verabreicht werden. Solche Suppositorien können dadurch hergestellt werden, daß man das Mittel mit einem geeigneten nicht reizenden Exzipiens vermischt, welches bei Raumtemperatur fest, bei der Rektaltemperatur jedoch flüssig ist und deshalb im Rektum schmilzt und so den Arzneistoff freigibt. Zu diesen Substanzen zählen Kakaobutter, Bienenwachs sowie Polyethylenglykole.

Die erfindungsgemäßen pharmazeutischen Zusammensetzungen können auch topisch verabreicht werden, insbesondere dann, wenn Flächen oder Organe, die einer topischen Anwendung leicht zugänglich sind, das Ziel der Behandlung bilden, darunter Augenerkrankungen, Hauterkrankungen oder Erkrankungen des unteren Verdauungstrakts. Geeignete topische Formulierungen lassen sich leicht für diese Flächen oder Organe herstellen.

Die topische Anwendung für den unteren Verdauungstrakt kann als Rektalsuppositorienformulierung wie oben beschrieben oder in Form eines geeigneter Darmeinlaufformulierung erfolgen. Topisch wirksame Transdermalpflaster können ebenfalls verwendet werden.

Für die topische Anwendung können die pharmazeutischen Zusammensetzungen als geeignete Salbe formuliert werden, die den wirksamen Bestandteil in einem oder mehreren Trägern suspendiert oder gelöst enthält. Zu Trägern für die topische Verabreichung der erfindungsgemäßen Verbindungen zählen Mineralöl, Paraffinöl, weißes Vaselin, Propylenglykol, Polyoxyethylen-Polyoxypropylen-Verbindung, emulgierendes Wachs und Wasser, was jedoch keine Einschränkung darstellen soll. Die pharmazeutischen Zusammensetzungen können jedoch auch als geeignete Lotion oder Creme, die die wirksamen Bestandteile in einem oder mehreren pharmazeutisch unbedenklichen Trägern suspendiert oder gelöst enthalten, formuliert werden. Zu geeigneten Trägern zählen Mineralöl, Sorbitanmonostearat, Polysorbat, Cetylesterwachs, Cetearylalkohol, 2-Octyldodecanol, Benzylalkohol und Wasser, was jedoch keine Einschränkung darstellen soll.

Zu pharmazeutischen Zusammensetzungen, auf die sich die vorliegende Verbindung erstreckt, zählen auch diejenigen, bei denen die therapeutisch wirksame Menge eines Wirkstoffs mit einer Verbindung, die zur Behandlung oder Vorbeugung von durch Modulation der PDE VII-Aktivität wie hierin beschrieben vermittelten bzw. damit assoziierten Erkrankungen, krankhaften Störungen und Leiden erforderlich ist, in einer zur systemischen Verabreichung geeigneten Dosierungsform bereitgestellt wird. Eine derartige pharmazeutische Zusammensetzung enthält den Wirkstoff in einer geeigneten flüssigen Form zur Abgabe durch: (1) Injektion oder Infusion, sei es intraarteriell, intra- oder transdermal, subkutan, intramuskulär, intraspinal, intrathecal oder intravenös, wobei der Wirkstoff (a) als gelöster Stoff in Lösung vorliegt, (b) in der offenen Phase einer Emulsion oder in der offenen Phase einer Emulsion mit Phasenumkehr, bei der sich die Phase bei Injektion oder Infusion umkehrt, wobei solche Emulsionen geeignete Emulgatoren enthalten, vorliegt, oder (c) in einer Suspension als suspendierter Feststoff in kolloidaler Form oder in Form feinster Teilchen vorliegt, wobei diese Suspension geeignete Suspendiermittel enthält, (2) Injektion oder Infusion in geeignete Körpergewebe oder -höhlen als Depot, wobei die Zusammensetzung den Wirkstoff lagert und anschließend zur systemischen Verteilung in Form einer Delayed-Release, Sustained-Release bzw. Controlled-Release freigibt, (3) Instillation, Inhalation oder Insufflation der pharmazeutischen Zusammensetzung in einer geeigneten festen Form in geeignete Körpergewebe oder -höhlen, wobei der Wirkstoff (a) in einem festen Implantat der Zusammensetzung vorliegt, das für die Freisetzung des Wirkstoffs in Form einer Delayed-Release, Sustained-Release bzw. Controlled-Release sorgt, (b) in einer teilchenförmigen Zusammensetzung, die in die Lunge eingeatmet wird, vorliegt, bzw. (c) in einer teilchenförmigen Zusammensetzung vorliegt, die in geeignete Körpergewebe oder -höhlen eingeblasen wird, wo die Zusammensetzung gewünschtenfalls für die Freisetzung des Wirkstoffs in Form einer Delayed-Release, Sustained-Release bzw. Controlled-Release bereitsteht, oder (4) Einnahme der pharmazeutischen Zusammensetzung in einer geeigneten festen oder flüssigen Form zur peroralen Abgabe des Wirkstoffs, wobei der Wirkstoff in einer festen Dosierungsform enthalten ist, oder (b) in einer flüssigen Dosierungsform enthalten ist.

Zu einzelnen Dosierungsformen der oben beschriebenen pharmazeutischen Zusammensetzungen zählen (1) Suppositorien als Spezialtyp eines Implantats, welche Grundlagen umfassen, die bei Raumtemperatur fest sind, jedoch bei Körpertemperatur schmelzen und so den Wirkstoff, den sie umfassen, langsam in das umgebende Körpergewebe abgeben, wo der Wirkstoff absorbiert wird und ein Transport erfolgt, so daß er systemisch verabreicht wird, (2) feste perorale Dosierungsformen der Gruppe (a) Oraltabletten, Kapseln, Caplets, Pastillen, Trochisken und mehrteilige Formen mit Delayed-Release-Freisetzung, (b) magensaftresistente Tabletten und Kapseln, die die Freisetzung und Absorption im Magen verhindern und so die Abgabe jenseits des Magens des behandelten Patienten ermöglichen, (c) Oraltabletten, Kapseln und feinteilige Formen mit Sustained-Release-Freigabe zur systemischen gesteuerten Ffreisetzung des Wirkstoffs über einen Zeitraum bis zu 24 Stunden, (d) rasch zerfallende Tabletten, (e) eingekapselte Lösungen, (f) Oralpasten, (g) ein Granulat, das in die bzw. in das Nahrungsmittel eines behandelten Patienten eingebracht wird, sowie (h) flüssige perorale Dosierungsformen aus der Gruppe der Lösungen, Suspensionen, Emulsionen, Emulsionen mit Phasenumkehr, Elexiere, Extrakte, Tinkturen und Konzentrate.

Zu pharmazeutischen Zusammensetzungen, auf die sich die vorliegende Verbindung erstreckt, zählen auch diejenigen, bei denen die therapeutisch wirksame Menge eines Wirkstoffs mit einer erfindungsgemäßen Verbindung, die zur Behandlung oder Vorbeugung von durch Modulation der PDE VII-Aktivität wie hierin beschrieben vermittelten bzw. damit assoziierten Erkrankungen, krankhaften Störungen und Leiden erforderlich ist, in einer Dosierungsform bereitgestellt wird, die sich für die lokale Verabreichung an einen behandelten Patienten eignet, wobei eine derartige pharmazeutische Zusammensetzung den Wirkstoff in einer geeigneten flüssigen Form enthält, um den Wirkstoff abzugeben durch (1) lokale Injektion oder Infusion, sei es intraarteriell, intraartikulär, intrachondrial, intrakostal, intrazystisch, intra- oder transdermal, intrafasciculär, intraligamentös, intramedullär, intramuskulär, intranasal, intraneural, intraoculär, d.h. opthalmische Verabreichung, intraossär, intrapelvin, intrapericardial, intraspinal, intrasternal, intrasynovial, intratarsal oder intrathecal, darunter auch Bestandteile, die für eine Delayed-Release-, Controlled-Release- bzw. Sustained-Release-Freisetzung des Wirkstoffs an diesem Lokus sorgen, wobei der Wirkstoff (a) als gelöster Stoff in Lösung vorliegt, (b) in der offenen Phase einer Emulsion oder in der offenen Phase einer Emulsion mit Phasenumkehr, bei der sich die Phase bei Injektion oder Infusion umkehrt, wobei solche Emulsionen geeignete Emulgatoren enthalten, vorliegt, oder (c) in einer Suspension als suspendierter Feststoff in kolloidaler Form oder in Form feinster Teilchen vorliegt, wobei diese Suspension geeignete Suspendiermittel enthält, (2) in einer Injektion oder Infusion als Depot enthalten ist zur Freisetzung des Wirkstoffs an den Lokus, wobei die Zusammensetzung den Wirkstoff lagert und anschließend an den Lokus in Form einer Delayed-Release, Sustained-Release bzw. Controlled-Release freigibt, wobei die Zusammensetzung auch Bestandteile beinhaltet, die sicherstellen, daß der Wirkstoff in erster Linie lokal wirkt und wenig systemisches Carry-over verursacht, oder wobei die pharmazeutische Zusammensetzung den Wirkstoff in einer geeigneten festen Form zur Abgabe des Inhibitors auf folgenden Weg enthält: (3) Instillation, Inhalation oder Insufflation an diesen Lokus, wobei der Wirkstoff enthalten ist in: (a) einem festen Implantat der Zusammensetzung, das an diesem Lokus implantiert wird, wobei die Zusammensetzung den Wirkstoff gegebenenfalls in Form einer Delayed-Release-, Sustained-Release- bzw. Controlled-Release-Freisetzung an den Lokus freigibt, (b) in einer teilchenförmigen Zusammensetzung, die in einen Lokus, darunter auch die Lunge, eingeatmet wird, bzw. (c) in einer teilchenförmigen Zusammensetzung, die in einen Lokus eingeblasen wird, wobei die Zusammensetzung Bestandteile beinhaltet, die sicherstellen, daß der Wirkstoff in erster Linie lokal wirkt und unwesentlich einem systemischen Carry-over unterliegt, sowie gegebenenfalls den Wirkstoff lokal in Form einer Delayed-Release-, Sustained-Release- bzw. Controlled-Relaise-Freigabe freisetzt. Zur ophthalmischen Verwendung lassen sich die pharmazeutischen Zusammensetzungen als mikronisierte Suspension in einer isotonischen sterilen Kochsalzlösung mit eingestelltem pH oder vorzugsweise als Lösungen in einer isotonischen sterilen Kochsalzlösung mit eingestelltem pH, mit oder ohne Konservierungsmittel wie Benzylalkoniumchlorid formulieren. Zur ophtalmischen Verwendung lassen sich die pharmazeutischen Zusammensetzungen auch in einer Salbe wie Vaselin formulieren.

Die erfindungsgemäßen pharmazeutischen Zusammensetzungen lassen auch mit einem Nasen-Aerosol oder durch Inhalation mit Verwendung eines Nebulisators, Trockenpulverinhalators oder Dosierinhalators verabreichen. Solche Zusammensetzungen werden nach Techniken, die in der Galenik gut bekannt sind, hergestellt, und können in Form von Lösungen in Kochsalzlösung mit Benzylalkohol oder anderen geeigneten Konservierungsmitteln, Resorptionsforderern zur Verbesserung der biologischen Verfügbarkeit, Fluorkohlenwasserstoffen und/oder anderen üblichen Solubilisierungsmitteln oder Dispergatoren hergestellt werden.

Wie bereits erwähnt können die erfindungsgemäßen Verbindungen einen zu behandelnden Patienten systemisch in Form einer pharmazeutischen Zusammensetzung in einer geeigneten flüssigen Form mittels Injektion oder Infusion verabreicht werden. Im Körper des Patienten befinden sich verschiedene Stellen und Organsysteme, die es der korrekt formulierten pharmazeutischen Zusammensetzung, sobald sie injiziert oder infundiert ist, gestatten, den gesamten Körper und alle Organsysteme des behandelten Patienten zu durchdringen. Bei einer Injektion handelt es sich um eine Einzeldosis der pharmazeutischen Zusammensetzung, die üblicherweise mittels einer Spritze in das betreffende Gewebe eingepreßt wird. Die häufigsten Arten der Injektion sind intramuskulär, intravenös und subkutan. Im Gegensatz dazu handelt es sich bei der Infusion um die langsame Einbringung der pharmazeutischen Zusammensetzung in das betroffene Gewebe. Die häufigste Art der Infusion ist die intravenöse Infusion. Zu weiteren Arten der Injektion oder Infusion zählen die intraarterielle, intra- oder transdermale (darunter auch subkutane) oder intraspinale, insbesondere intratekale, Injektion oder Infusion. In diesen flüssigen pharmazeutischen Zusammensetzungen kann der Wirkstoff als gelöster Stoff in Lösung vorliegen. Dies stellt den häufigsten und am stärksten bevorzugten Typ einer solchen Zusammensetzung dar, es ist jedoch ein Wirkstoff in einer Salzform erforderlich, die eine einigermaßen gute Löslichkeit in Wasser aufweist. Das mit Abstand am stärksten bevorzugte Lösungsmittel für solche Zusammensetzungen ist Wasser (oder Kochsalzlösung). Gelegentlich können übersättigte Lösungen verwendet werden, diese sind jedoch problematisch in bezug auf ihre Stabilität und daher für den alltäglichen Gebrauch unpraktisch.

Falls es nicht möglich ist, eine bevorzugte Verbindung in einer Form zu erhalten, die die erforderliche Löslichkeit in Wasser aufweist, wie dies manchmal der Fall ist, kann der Durchschnittsfachmann mit seinen Fähigkeiten eine Emulsion herstellen, wobei es sich um eine Dispersion von kleinen Tröpfchen einer Flüssigkeit, der offenen oder inneren Phase, in einer zweiten Flüssigkeit, der geschlossenen oder äußeren Phase, mit der diese unmischbar ist, handelt. Die beiden Flüssigkeiten werden durch pharmazeutisch unbedenkliche Emulgatoren in emulgiertem Zustand gehalten. Handelt es sich bei dem Wirkstoff um ein wasserunlösliches Öl, kann er daher in einer Emulsion, bei der er die offene Phase bildet, verabreicht werden. Ist der Wirkstoff wasserunlöslich, jedoch in einem mit Wasser unmischbaren Lösungsmittel löslich, kann ebenfalls eine Emulsion verwendet werden. Obwohl der Wirkstoff am häufigsten als offene oder innere Phase einer sogenannten Öl-in-Wasser-Emulsion verwendet würde, könnte er auch als offene oder innere Phase einer Emulsion mit Phasenumkehr, die üblicherweise als Wasser-in-Öl-Emulsion bezeichnet wird, verwendet werden. Hier ist der Wirkstoff wasserlöslich und könnte als einfache wäßrige Lösung verabreicht werden. Solche Emulsionen mit Phasenumkehr invertieren jedoch bei Injektion oder Infusion in ein wäßriges Medium, wie das Blut, und bieten den Vorteil eines rascheren und wirksameren Dispergierens des Wirkstoffs in dieses wäßrige Medium als bei Verwendung einer wäßrigen Lösung. Emulsionen mit Phasenumkehr werden mit fachbekannten geeigneten pharmazeutisch unbedenklichen Emulgatoren hergestellt.

Ist der Wirkstoff beschränkt wasserlöslich, so kann er auch als suspendierter Feststoff in kolloidaler oder feinteiliger Form in einer Suspension, die unter Verwendung geeigneter pharmazeutisch unbedenklicher Suspendiermittel hergestellt wird, verabreicht werden. Die den Wirkstoff enthaltenden suspendierten Feststoffe können auch als Zusammensetzungen mit Delayed-Release-, Sustained-Release-, bzw. Controlled-Release-Freigabe formuliert werden.

Obwohl die systemische Verabreichung am häufigsten durch Injektion oder Infusion einer Flüssigkeit erfolgt, existieren viele Situationen, in denen es vorteilhaft oder sogar erforderlich ist, den Wirkstoff als Feststoff abzugeben. Die systemische Verabreichung von Feststoffen wird durch Instillation, Inhalation oder Insufflation einer pharmazeutischen Zusammensetzung in geeigneter fester Form, die den Wirkstoff enthält, durchgeführt. Bei der Instillation des Wirkstoffs kann ein festes Implantat der Zusammensetzung in geeignete Körpergewebe oder -höhlen eingesetzt werden. Das Implantat kann eine Matrix aus biologisch kompatiblen und biologisch abbaubaren Substanzen enthalten, in der Teilchen eines festen Wirkstoffs dispergiert sind, oder in der möglicherweise Tröpfchen oder isolierte Zellen eines flüssigen Wirkstoffs eingeschlossen sind. Die Matrix soll vom Körper möglichst abgebaut und vollständig resorbiert werden. Die Zusammensetzung der Matrix wird auch bevorzugt so ausgewählt, daß der Wirkstoff über längere Zeiträume, sogar mehrere Monate, in Form einer Controlled-Release-, Sustained-Release- bzw. Delayed-Release-Freisetzung abgegeben wird.

Der Ausdruck "Implantat" bezieht sich meistens auf eine feste wirkstoffhaltige pharmazeutische Zusammensetzung, während der Ausdruck "Depot" üblicherweise eine flüssige wirkstoffhaltige pharmazeutische Zusammensetzung bedeutet, die in einem beliebigen geeigneten Körpergewebe oder einer beliebigen geeigneten Körperhöhle abgelegt wird und so ein Reservoir oder einen Pool bildet, der langsam in die umgebenden Gewebe und Organe wandert und schließlich und endlich systemisch verteilt wird. Diese Unterscheidungen werden in der Fachwelt jedoch nicht immer streng gehandhabt und es wird daher vorgesehen, daß sich der Umfang der vorliegenden Erfindung auf flüssige Implantate und feste Depots sowie sogar jeweils feste und flüssige Mischformen erstreckt. Suppositorien können als eine Art Implantat aufgefaßt werden, da sie Grundlagen enthalten, die bei Raumtemperatur fest sind, jedoch bei der Körpertemperatur eines Patienten schmelzen und so den Wirkstoff, mit dem sie ausgestattet sind, langsam in das umgebende Gewebe des Körpers des Patienten freigeben, wo der Wirkstoff resorbiert und abtransportiert wird, und so systemisch verabreicht wird.

Die systemische Verabreichung läßt sich auch mittels Inhalation oder Insufflation eines Pulvers durchführen, d.h. einer teilchenförmigen wirkstoffhaltigen Zusammensetzung. Zum Beispiel kann der Wirkstoff in Pulverform mit üblichen Geräten zur Aerosolbildung teilchenförmiger Formulierungen in die Lunge eingeatmet werden. Der Wirkstoff kann als teilchenförmige Formulierung auch durch Insufflation verabreicht werden, d.h. durch einfaches Stäuben oder mit üblichen Geräten zur Aerosolbildung von teilchenförmigen Formulierungen in geeignete Körpergewebe oder -höhlen geblasen oder anderweitig dispergiert werden. Diese teilchenförmigen Zusammensetzungen können ebenfalls nach gut bekannten Prinzipien und mit bekannten Materialien so formuliert werden, daß man zu einem Wirkstoff mit Delayed-Release-, Sustained-Release- bzw. Controlled-Release-Freigabe gelangt.

Zu weiteren Möglichkeiten der systemischen Verabreichung, bei denen die erfindungsgemäßen Wirkstoffe entweder in flüssiger oder in fester Form verwendet werden, zählen der transdermale, intranasale und ophthalmische Verabreichungsweg. Insbesondere können Transdermalpflaster nach in der Arzneistoffabgabe bekannten Techniken hergestellt und auf die Haut des zu behandelnden Patienten aufgebracht werden, wonach der Wirkstoff aufgrund seiner formulierten Löslichkeitseigenschaften durch die Epidermis und in die Dermisschichten der Haut des Patienten wandert, wo er als Teil der allgemeinen Zirkulation des Patienten aufgenommen wird und schließlich und endlich zu einer systemischen Verteilung des Wirkstoffs über eine gewünschte, längere Zeitdauer führt. Dazu zählen auch Implantate, die unter die Epidermisschicht der Haut gegeben werden, d.h. zwischen die Epidermis und die Dermis der Haut des behandelten Patienten. Ein derartiges Implantat wird gemäß gut bekannter Prinzipien und Materialien, die häufig bei dieser Abgabetechnik verwendet werden, formuliert, und kann auf solche Art und Weise hergestellt werden, daß der Wirkstoff nach dem Prinzip der Controlled-Release-, Sustained-Release- bzw. Delayed-Release-Freisetzung in die systemische Zirkulation des Patienten abgegeben wird. Derartige subepidermalen (subkutikulären) Implantate sind genauso leicht wie Transdermalpflaster einzusetzen und bieten die gleiche wirksame Abgabe, jedoch ohne dem Abbau, der Schädigung oder der zufälligen Entfernung ausgesetzt zu sein, die davon herrührt, daß das Pflaster auf der äußersten Schicht der Haut des Patienten exponiert ist.

In der obigen Beschreibung der pharmazeutischen Zusammensetzungen, die eine bevorzugte Verbindung enthalten, wurden die gleichwertigen Ausdrücke "Verabreichung", "Verabreichung von", "Verabreichen" und "ein (e)...verabreichen", in bezug auf diese pharmazeutischen Zusammensetzungen verwendet. Diese Ausdrücke sollen im vorliegenden Zusammenhang bedeuten, daß einen behandlungsbedürftigen Patienten eine erfindungsgemäße pharmazeutische Zusammensetzung auf einem beliebigen der hier beschriebenen Verabreichungswege zur Verfügung gestellt wird, wobei es sich bei dem Wirkstoff um eine bevorzugte Verbindung oder ein Prodrug, ein Derivat oder einen Metaboliten hiervon handelt, das bzw. der sich zur Behandlung einer durch Modulation der PDE VII-Aktivität vermittelten oder hiermit assoziierten Erkrankung, krankhaften Störung oder eines derartigen Leiden bei diesem Patienten eignet. Die vorliegende Erfindung erstreckt sich daher auf eine beliebige andere Verbindung, die bei Verabreichung an einen Patienten fähig ist, eine bevorzugte Verbindung direkt oder indirekt zur Verfügung zu stellen. Solche Verbindungen sind als Prodrugs bekannt, und es existieren viele etablierte Vorgehensweisen zur Herstellung solcher Prodrug-Formen der bevorzugten Verbindungen.

Die Dosis bzw. Dosierung der bei der Behandlung oder Vorbeugung einer durch Modulation der PDE VII-Aktivität vermittelten bzw. damit assoziierten Erkrankung, krankhaften Störung bzw. eines derartigen Leidens hängt von verschiedenen Faktoren wie der Art des Inhibitors, der Größe des Patienten, dem Behandlungsziel, der Art der zu behandelnden Pathologie, der jeweils verwendeten pharmazeutischen Zusammensetzung sowie den Beobachtungen und Schlußfolgerungen des behandelnden Arztes ab.

Bei einer oralen Dosierungsform, z.B. einer Tablette oder Kapsel, liegen geeignete Dosismengen der erfindungsgemäßen Verbindungen zwischen ungefähr 0,1 µg Wirkstoff/kg und ungefähr 50,0 mg Wirkstoff/kg Körpergewicht und Tag, vorzugsweise zwischen ungefähr 5,0 µg Wirkstoff/kg und ungefähr 5,0 mg Wirkstoff/kg Körpergewicht und Tag, stärker bevorzugt zwischen ungefähr 10,0 µg Wirkstoff/kg und ungefähr 1,0 mg Wirkstoff/kg Körpergewicht und Tag, am stärksten bevorzugt zwischen ungefähr 20,0 µg Wirkstoff/kg und ungefähr 0,5 mg Wirkstoff/kg Körpergewicht und Tag.

Wird die Dosierungsform topisch an die Bronchien und die Lunge verabreicht, z.B. mittels Pulverinhalator oder Nebulisator, so liegen geeignete Dosismengen der Verbindungen zwischen ungefähr 0,001 µg Wirkstoff/kg und ungefähr 10,0 mg Wirkstoff/kg Körpergewicht und Tag, vorzugsweise zwischen ungefähr 0,5 µg Wirkstoff/kg und ungefähr 0,5 mg Wirkstoff/kg Körpergewicht und Tag, stärker bevorzugt zwischen 1,0 µg Wirkstoff/kg und ungefähr 0,1 mg Wirkstoff/kg Körpergewicht und Tag, am stärksten bevorzugt zwischen ungefähr 2,0 µg Wirkstoff/kg und ungefähr 0,05 mg Wirkstoff/kg Körpergewicht und Tag.

Um den Bereich der oralen Tagesdosis, die wie oben beschrieben verwendet werden könnte, zu erläutern und unter Zuhilfenahme eines typischen Körpergewichts von 10 kg und 100 kg liegen geeignete Dosismengen der erfindungsgemäßen Verbindungen zwischen ungefähr 1,0 - 10,0 µg und 500,0 - 5000,0 mg des Wirkstoffs mit einer bevorzugten Verbindung pro Tag, vorzugsweise zwischen ungefähr 50,0 und 500,0 µg und 50,0 - 500,0 mg des Wirkstoffs mit einer bevorzugten Verbindung pro Tag, stärker bevorzugt zwischen ungefähr 100,0 - 1000,0 µg und 10,0 - 100,0 mg eines Wirkstoffs mit einer bevorzugten Verbindung pro Tag, am stärksten bevorzugt zwischen ungefähr 200,0 - 20000 µg und ungefähr 5,0 - 500 mg des Wirkstoffs mit einer bevorzugten Verbindung pro Tag. Diese Dosierbereiche stellen Gesamtdosismengen des Wirkstoffs pro Tag für einen bestimmten Patienten dar. Wie oft eine Dosis pro Tag verabreicht wird, hängt von pharmakologischen und pharmakokinetischen Faktoren wie der Halbwertszeit des Wirkstoffs, welche seine Katabolisierungsgeschwindigkeit und Clearance widerspiegelt, sowie dem minimalen und optimalen Blutplasmaspiegel bzw. anderen Körperflüssigkeitsspiegeln des Wirkstoffs in einem Patienten, die für eine therapeutische Wirksamkeit erforderlich sind, ab.

Bei der Festsetzung der Anzahl Dosen pro Tag und der Wirkstoffmenge pro Dosis, die verabreicht werden, müssen auch zahlreiche andere Faktoren in Betracht gezogen werden. Ein weiterer derartiger Faktor ist nicht zuletzt die jeweilige Reaktion des behandelten Patienten. So werden zum Beispiel bei Verwendung des Wirkstoffs zur Behandlung oder Vorbeugung von Asthma bei topischer Verabreichung über Aerosol-Inhalation in die Lungen ein bis vier Dosen, die aus Betätigungen eines Abgabegeräts bestehen, d.h. "Sprühstößen" eines Inhalators, pro Tag verabreicht, wobei jede Dosis ungefähr 50,0 µg bis ungefähr 10,0 mg Wirkstoff enthält.

Die Erfindung betrifft weiterhin auch Arzneimittel mit mindestens einer erfindungsgemäßen Verbindung und/oder ihren pharmazeutisch verwendbaren Derivaten, Solvaten und Stereoisomeren, deren Mischungen in allen Verhältnissen, sowie gegebenenfalls Exzipientien und/oder Hilfsstoffen.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und/oder durch Kristallisation
Massenspektrometrie (MS) (electron impact ionization) M⁺
FAB (fast atom bombardment)(M+H)⁺

### Beispiel 1

1.1 Zu einer Lösung von 1,05 g p-Toluidin in 2,4 ml konz. HCl tropft man bei 0° eine Lösung von 0,68 g Natriumnitrit in 20 ml Wasser. Die Lösung enthält 1,51 g 4-Methyl-phenyldiazoniumhydrochlorid und wird sofort umgesetzt.
1.2 Eine Lösung von 55 g 2-(Isobutyryl)-essigsäureethylester ("AA") in 210 ml Toluol wird mit 30 g Malonsäuredinitril, 3,3 g Ammoniumacetat und 4 ml Essigsäure versetzt und 1 Stunde am Wasserabscheider bei 130° gekocht. Man arbeitet wie üblich auf und erhält 41,0 g 4,4-Dicyan-3-isopropyl-but-3-en-säureethylester ("AB"), Siedepunkt 95-99° bei 0,4 mbar.
1.3 Zu einer Lösung von 2,0 g "AB" in 52 g Ethanol gibt man 3,45 g Natriumacetat, rührt 10 Minuten, kühlt auf 0°, und gibt die kalte Lösung mit 1,51 g 4-Methyl-phenyldiazoniumhydrochlorid zu. Man rührt 1 Stunde bei 0° nach. Man alkalisiert mit Ammoniaklösung, arbeitet wie üblich auf und erhält 3,1 g 5-Cyan-6-imino-4-isopropyl-1-p-tolyl-1,6-dihydro-pyridazin-3-carbonsäureethylester ("AC") als Öl.
1.4 Zu einer Lösung von 3,1 g "AC" in 38 ml Essigsäure (100%) gibt man bei 14° portionsweise 4,1 g Zinkpulver. Man rührt 1 Stunde nach, filtriert, wäscht mit wenig Eisessig und wenig Dichlormethan. Das Filtrat wird mit 400 ml Wasser versetzt, 30 Minuten nachgerührt, abgetrennt und mit Wasser gewaschen. Man erhält nach Trocknen 2,6 g 5-Amino-4-cyan-3-isopropyl-1-p-tolyl-1H-pyrrol-2-carbonsäureethylester ("AD"), F. 156°.
1.5 Eine Lösung von 800 mg "AD" in 12 ml Ameisensäure (98-100%) wird 2 Stunden bei 105° gerührt. Das Lösungsmittel wird abgetrennt und der Rückstand aus Isopropanol kristallisiert. Man erhält 600 mg 5-Isopropyl-4-oxo-7-p-tolyl-4,7-dihydro-3*H*-pyrrolo[2,3-*d*]-pyrimidin-6-carbonsäureethylester.

Analog erhält man durch Umsetzung von
5-Amino-4-cyan-3-methyl-1-(3-chlorphenyl)-1H-pyrrol-2-carbonsäureethylester,
5-Amino-4-cyan-3-methyl-1-(2-chlorphenyl)-1 H-pyrrol-2-carbonsäureethylester,
5-Amino-4-cyan-3-methyl-1-(2-fluorphenyl)-1H-pyrrol-2-carbonsäureethylester,
5-Amino-4-cyan-3-propyl-1-(2-chlorphenyl)-1H-pyrrol-2-carbonsäureethylester,
5-Amino-4-cyan-3-methyl-1-(4-chlorphenyl)-1H-pyrrol-2-carbonsäureethylester,
5-Amino-4-cyan-3-methyl-1-(2-chlorphenyl)-1H-pyrrol-2-carbonsäuremethylester,
5-Amino-4-cyan-3-methyl-1-phenyl-1 H-pyrrol-2-carbonsäuremethylester,
5-Amino-4-cyan-3-methyl-1-(2-thienyl)-1 H-pyrrol-2-carbonsäuremethylester,
mit Ameisensäure die nachstehenden Verbindungen
5-Methyl-4-oxo-7-(3-chlorphenyl)-4,7-dihydro-3*H*-pyrrolo[2,3-*d*]-pyrimidin-6-carbonsäureethylester,
5-Methyl-4-oxo-7-(2-chlorphenyl)-4,7-dihydro-3*H*-pyrrolo[2,3-*d*]-pyrimidin-6-carbonsäureethylester,
5-Methyl-4-oxo-7-(2-fluorphenyl)-4,7-dihydro-3*H*-pyrrolo[2,3-*d*]-pyrimidin-6-carbonsäureethylester,
5-Propyl-4-oxo-7-(2-chlorphenyl)-4,7-dihydro-3*H*-pyrrolo[2,3-*d*]-pyrimidin-6-carbonsäureethylester,
5-Methyl-4-oxo-7-(4-chlorphenyl)-4,7-dihydro-3*H*-pyrrolo[2,3-*d*]-pyrimidin-6-carbonsäureethylester,
5-Methyl-4-oxo-7-(2-chlorphenyl)-4,7-dihydro-3*H*-pyrrolo[2,3-*d*]-pyrimidin-6-carbonsäuremethylester,
5-Methyl-4-oxo-7-phenyl-4,7-dihydro-3*H*-pyrrolo[2,3-*d*]-pyrimidin-6-carbonsäuremethylester,
5-Methyl-4-oxo-7-(2-thienyl)-4,7-dihydro-3*H*-pyrrolo[2,3-*d*]-pyrimidin-6-carbonsäuremethylester.

Die folgenden Beispiel betreffen pharmazeutische Zubereitungen:

### Beispiel A: Injektionsgläser

Eine Lösung von 100 g eines erfindungsgemäßen Wirkstoffes und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines erfindungsgemäßen Wirkstoffes mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines erfindungsgemäßen Wirkstoffes, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines erfindungsgemäßen Wirkstoffes mit 99,5 g Vaselin unter aseptischen Bedingungen.

### Beispiel E: Tabletten

Ein Gemisch von 1 kg Wirkstoff, 4 kg Laktose, 1,2 kg Kartoffelstärke, 0,2 kg Talk und 0,1 kg Magnesiumstearat wird in üblicher Weise so zu Tabletten verpreßt, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen ausgewählt aus der Gruppe
5-Isopropyl-4-oxo-7-p-tolyl-4,7-dihydro-3*H*-pyrrolo[2,3-*d*]-pyrimidin-6-carbonsäureethylester,
5-Methyl-4-oxo-7-(3-chlorphenyl)-4,7-dihydro-3*H*-pyrrolo[2,3-*d*]-pyrimidin-6-carbonsäureethylester,
5-Methyl-4-oxo-7-(2-chlorphenyl)-4,7-dihydro-3*H*-pyrrolo[2,3-*d*]-pyrimidin-6-carbonsäureethylester,
5-Methyl-4-oxo-7-(2-fluorphenyl)-4,7-dihydro-3*H*-pyrrolo[2,3-*d*]-pyrimidin-6-carbonsäureethylester,
5-Propyl-4-oxo-7-(2-chlorphenyl)-4,7-dihydro-3*N*-pyrrolo[2,3-*d*]-pyrimidin-6-carbonsäureethylester,
5-Methyl-4-oxo-7-(4-chlorphenyl)-4,7-dihydro-3*H*-pyrrolo[2,3-*d*]-pyrimidin-6-carbonsäureethylester,
5-Methyl-4-oxo-7-(2-chlorphenyl)-4,7-dihydro-3*H*-pyrrolo[2,3-*d*]-pyrimidin-6-carbonsäuremethylester,
5-Methyl-4-oxo-7-phenyl-4,7-dihydro-3*H*-pyrrolo[2,3-*d*]-pyrimidin-6-carbonsäuremethylester,
5-Methyl-4-oxo-7-(2-thienyl)-4,7-dihydro-3*H*-pyrrolo[2,3-*d*]-pyrimidin-6-carbonsäuremethylester,
sowie ihre pharmazeutisch verwendbaren Salze, Solvate und Stereoisomere, einschließlich deren Mischungen in allen Verhältnissen.

2. Verbindungen nach Anspruch 1 als Phosphodiesterase VII-Inhibitoren.

3. Verbindungen nach Anspruch 1 und ihre physiologisch unbedenklichen Salze und Solvate zur Behandlung oder Prophylaxe von Krankheiten, die durch Verbindungen mit PDE VII-inhibitorischer Aktivität bekämpft oder beeinflußt werden können.

4. Arzneimittel mit mindestens einer Verbindung nach Anspruch 1 und/oder ihren pharmazeutisch verwendbaren Salzen, Solvaten und Stereoisomeren, einschließlich deren Mischungen in allen Verhältnissen, sowie gegebenenfalls Träger- und/oder Hilfsstoffe.

5. Verwendung von Verbindungen nach Anspruch 1 und/oder ihren physiologisch unbedenklichen Salzen oder Solvaten zur Herstellung eines Arzneimittels zur Behandlung eines Patienten, der an einer durch das PDE VII-Isozym, in seiner Rolle bei der Regulierung der Aktivierung und Degranulation von menschlichen Eosinophilen, vermittelten Erkrankung oder Störung leidet.

6. Verwendung nach Anspruch 5 von Verbindungen nach Anspruch 1 und/oder ihren physiologisch unbedenklichen Salzen oder Solvaten zur Herstellung eines Arzneimittels zur Bekämpfung von allergischen Krankheiten, Asthma, chronischer Bronchitis, atopischer Dermatitis, Psoriasis und anderer Hautkrankheiten, entzündlichen Erkrankungen, Autoimmunerkrankungen, wie z.B. rheumatoide Arthritis, multiple Sklerose, Morbus Crohn, Diabetes mellitus oder ulzerative Kolitis, Osteoporose, Transplantatabstoßungsreaktionen, Kachexie, Tumorwachstum oder Tumormetastasen, Sepsis, Gedächtnisstörungen, Atherosklerose und AIDS.

7. Verwendung nach Anspruch 5 oder 6 einer Verbindung nach Anspruch 1 zur Herstellung eines Arzneimittels zur Behandlung oder Vorbeugung von einer oder mehreren aus der Gruppe der folgenden Erkrankungen, krankhaften Störungen und Leiden:
Asthma jeglicher Art, Ätiologie oder Pathogenese, oder Asthma aus der Gruppe atopisches Asthma, nichtatopisches Asthma, allergisches Asthma, IgE-vermitteltes atopisches Asthma, Bronchialasthma, essentielles Asthma, Primärasthma, durch pathophysiologische Störungen hervorgerufenes endogenes Asthma, durch Umweltfaktoren hervorgerufenes exogenes Asthma, essentielles Asthma unbekannter oder inapparenter Ursache, nichtatopisches Asthma, bronchitisches Asthma, emphysematöses Asthma, durch Belastung induziertes Asthma, Berufsasthma, durch Bakterien-, Pilz-, Protozoen- oder Virusinfektion hervorgerufenes infektallergisches Asthma, nichtallergisches Asthma, inzipientes Asthma, "wheezy infant syndrome";
chronische oder akute Bronchokonstriktion, chronische Bronchitis, Obstruktion der kleinen Atemwege sowie Emphysem;
obstruktive oder entzündliche Atemwegserkrankung jeglicher Art, Ätiologie oder Pathogenese, oder eine obstruktive oder entzündliche Atemwegserkrankung aus der Gruppe Asthma; Staublunge, chronische eosinophile Pneumonie; chronischer obstruktive pulmonaler Krankheit (COPD); COPD inklusive chronische Bronchitis, Lungenemphysem oder damit assoziierte Atemnot, durch irreversible, fortschreitende Obstruktion der Atemwege **gekennzeichnet**e COPD, Schocklunge (adult respiratory distress syndrome, ARDS) sowie Verschärfung der Überempfindlichkeit der Atemwege aufgrund Therapie mit anderen Arzneistoffen;
Staublunge jeglicher Art, Ätiologie oder Pathogenese, oder Staublunge aus der Gruppe Aluminose oder Aluminiumstaublunge, Anthrakose(-Asthma), Asbestose oder Asbeststaublunge, Chalikose oder Kalkstaublunge, durch Einatmen von Straußenfedernstaub verursachte Ptilose, durch Einatmung von Eisenteilchen verursachte Siderose, Silikose oder Steinstaublunge, Byssinose oder Baumwollstaubpneumokoniose sowie Talkpneumokoniose;
Bronchitis jeglicher Art, Atiologie oder Pathogenese, oder Bronchitis aus der Gruppe akute Bronchitis, akute laryngotracheale Bronchitis, durch Erdnüsse ausgelöste Bronchitis, Bronchialkatarrh, kruppöse Bronchitis, Bronchitis ohne Auswurf, infektiöse Asthmabronchitis, Bronchitis mit Auswurf, Staphylokokken- oder Streptokokkenbronchitis; sowie Vesikulärbronchitis;
Bronchiektasie jeglicher Art, Ätiologie oder Pathogenese, oder Bronchiektasie aus der Gruppe zylindrische Bronchiektasie, sackförmige Bronchiektasie, spindelförmige Bronchiektasie, Bronchiolendilatation, zystische Bronchiektasie, Bronchiektasie ohne Auswurf, sowie follikuläre Bronchiektasie;
jahreszeitlich bedingte allergische Rhinitis, perenniale allergische Rhinitis, oder Sinusitis jeglicher Art, Ätiologie oder Pathogenese, oder Sinusitis aus der Gruppe eitriger oder nichteitriger Sinusitis, akute oder chronische Sinusitis, Ethmoiditis, Stirnhöhlenentzündung, Kieferhöhlenentzündung oder Sphenoiditis;
rheumatoide Arthritis jeglicher Art, Ätiologie oder Pathogenese, oder rheumatoide Arthritis aus der Gruppe akute Arthritis, akute Gichtarthritis, primär-chronische Polyarthritis, Osteoarthrose, Infektarthritis, Lyme-Arthritis, progrediente Arthritis, Arthritis psoriatica, sowie Spondylarthritis;
Gicht sowie mit Entzündung assoziierte Fieber bzw, mit Entzündung assoziierte Schmerz;
eine mit Eosinophilen in Zusammenhang stehende krankhafte Störung jeglicher Art, Ätiologie oder Pathogenese, oder eine mit Eosinophilen in Zusammenhang stehende krankhafte Störung aus der Gruppe Eosinophilie, eosinophiles Lungeninfltrat, Löffler-Syndrom, chronische eosinophile Pneumonie, tropische Lungeneosinophilie, bronchopneumonische Aspergillose, Aspergillom, eosinophiles Granulom, allergische granulomatöse Angiitis bzw. Churg-Strauss-Syndrom, Polyarteriitis nodosa (PAN), sowie systemische Vasculitis necroticans;
atopische Dermatitis, allergische Dermatitis, oder allergisches oder atopisches Ekzem;
Nesselsucht jeglicher Art, Ätiologie oder Pathogenese, oder Nesselsucht aus der Gruppe immunbedingte Nesselsucht,
Komplement-bedingte Nesselsucht, durch Nesselsucht auslösendes Material induzierte Nesselsucht, durch physikalische Reize ausgelöste Nesselsucht, durch Streß ausgelöste Nesselsucht, idiopatische Nesselsucht, akute Nesselsucht, chronische Nesselsucht, angioneurotisches Ödem, Urticaria cholinergica, Kälteurtikaria in ihrer autosomal-dominanten Form oder in ihrer erworbenen Form, Kontakturtikaria, Urticaria gigantean sowie Papelurtikaria;
Konjunktivitis jeglicher Art, Ätiologie oder Pathogenese, oder Konjunktivitis aus der Gruppe Conjunctivitis actinica, akute katarrhalische Konjunktivitis, akute contagiöse Konjunktivitis, allergische Konjunktivitis, atopische Konjunktivitis, chronische katarrhalische Konjunktivitis, eitrige Konjunktivitis sowie Frühjahrskonjunktivitis; Uveitis jeglicher Art, Ätiologie oder Pathogenese oder Uveitis aus der Gruppe Entzündung der ganzen Uvea oder eines Teils davon, Uveitis anterior, Iritis, Cyclitis, Iridocyclitis, granulomatöse Uveitis, nichtgranulomatöse Uveitis, phakoantigene Uveitis, Uveitis posterior, Choroiditis sowie Choriorethinitis;
Schuppenflechte;
multiple Sklerose jeglicher Art, Ätiologie oder Pathogenese, oder multiple Sklerose aus der Gruppe primär progrediente multiple Sklerose sowie multiple Sklerose mit schubweisem Verlauf und Neigung zu Remissionen;
Autoimmun-/Entzündungserkrankungen jeglicher Art, Ätiologie oder Pathogenese, oder eine Autoimmun-/Entzündungserkrankung aus der Gruppe autoimmunhämatologische Störungen, hämolytische Anämie, aplastische Anämie, aregenerative Anämie, idiopatische thrombozytopene Purpura, systemischer Lupus erythematosus, Polychondritis, Skleroderm, Wegener-Granulomatose, Lichtkrankheit, chronisch-aktive Hepatitis, Myasthenia gravis, Stevens-Johnson-Syndrom, idiopathische Sprue, Autoimmun-Reizkolonerkrankungen, Colitis ulcerosa, Morbus Crohn, endokrine Opthamopathy, Basedow-Krankheit, Sarkoidose, Alveolitis, chronische Hypersensitivitätspneumonitis, primär biliäre Zirrhose, Insulinmangeldiabetes oder Typ 1 Diabetes mellitus, Uveitis anterior, granulomatöse Uveitis oder Uveitis posterior, Keratoconjunctivitis sicca, Keratoconjunctivitis epidemica, (diffuse) interstitielle Lungenfibrose, Lungenzirrhose, Mukoviszidose, Arthritis psoriatica, Glomerulonephritis mit und ohne Nephrose, akute Glomerulonephritis, idiopathische Nephrose, Minimal-Change-Nephropathie, entzündliche/ hyperproliferative Hauterkrankungen, Schuppenflechte, atopische Dermatitis, Kontaktdermatitis, allergische Kontaktdermatitis, familiärer gutartiger Pemphigus, Pemphigus erythematosus, Pemphigus foliaceus sowie Pemphigus vulgaris;
Vorbeugung einer Fremdtransplantatabstoßung nach Organtransplantation,
Reizdarm (inflammatory bowel disease, IBD) jeglicher Art, Ätiologie oder Pathogenese, oder Reizdarm aus der Gruppe ulzerative Kolitis (UC), kollagenöse Kolitis, Colitis polyposa, transmurale Kolitis sowie Morbus Crohn (CD);
septischer Schock jeglicher Art, Ätiologie oder Pathogenese, oder septischer Schock aus der Gruppe Nierenversagen, akutes Nierenversagen, Kachexie, Malariakachexie, hypophysäre Kachexie, uremämische Kachexie, Herzkachexie, Cachexia suprarenalis bzw. Addison-Krankheit, karzinomatöse Kachexie sowie Kachexie auf Grund von Infektion durch Human Immunodeficiency Virus (HIV); Leberschädigung;
pulmonaler Hochdruck sowie durch Sauerstoffmangel hervorgerufener pulmonaler Hochdruck;
Knochenschwunderkrankungen, primäre Osteoporose und sekundäre Osteoporose;
krankhafte Störungen des Zentralnervensystems jeglicher Art, Ätiologie oder Pathogenese, oder eine krankhafte Störung des Zentralnervensystems aus der Gruppe Depression, Morbus Parkinson, Lern- und Gedächtnisstörungen, tardive Dyskinesie, Drogenabhängigkeit, arteriosklerotische Demenz, sowie Demenz als Begleiterscheinung von Chorea Huntington, Morbus Wilson, Paralysis agitans sowie Thalamusatrophien;
Infektionen, insbesondere Virusinfektionen, wobei diese Viren die Produktion von TNF-α in ihrem Wirt erhöhen oder wobei diese Viren gegenüber Hinaufregulierung von TNF-α in ihrem Wirt empfindlich sind, so daß ihre Replikation oder andere wichtigen Aktivitäten behindert werden, darunter Viren aus der Gruppe HIV-1, HIV-2 und HIV-3, Zytomegallevirus, CMV; Grippe, Adenoviren und Herpesviren, darunter Herpes zoster und Herpes simplex;
Hefe- und Pilzinfektionen, wobei diese Hefen und Pilze gegenüber Hinaufregulierung durch TNF-α empfindlich sind oder die TNF-α-Produktion in ihrem Wirt auslösen, z.B. Pilzmeningitis, insbesondere bei gemeinsamer Verabreichung mit anderen Arzneistoffen der Wahl zur Behandlung systemischer Hefe- und Pilzinfektionen, darunter den Polymycinen, z.B. Polymycin B, Imidazolen, z.B. Clotrimazol, Econazol, Miconazol und Ketoconazol, den Triazolen, z.B. Fluconazol und Itranazol, sowie den Amphotericinen, z.B. Amphotericin B und liposomales Amphotericin B, was jedoch keine Einschränkung darstellen soll.
Ischämie-Reperfusionsschädigung, Autoimmundiabetes, retinale Autoimmunität, chronische lymphozytische Leukämie, HIV-Infektionen, Lupus erythematosus, Nieren- und
Hamleitererkrankungen, krankhafte Urogenital- und Gastrointestinalstörungen, sowie Prostataerkrankungen.

8. Verwendung nach Anspruch 5, 6 oder 7 einer Verbindung nach Anspruch 1 zur Herstellung eines Arzneimittels zur Behandlung von (1) Entzündungserkrankungen und -leiden, inklusive Gelenkentzündung, rheumatoide Arthritits, rheumatoide Spondylitis, Osteoarthritis, Reizdarm, ulzerative Kolitis, chronische Glomerulonephritis, Dermatitis sowie Morbus Crohn, (2) Erkrankungen und Leiden der Atemwege, inklusive Asthma, Schocklunge, chronische Pulmonitis, Bronchitits, chronische obstruktive Atemwegserkrankung sowie Silikose, (3) Infektionskrankheiten und -leiden inkluisve Sepsis, septischer Schock, endotoxischer Schock, gramnegative Sepsis, toxisches Schocksyndrom, durch Bakterien-, Virus- oder Pilzinfektionen hervorgerufenes Fieber bzw. Myalgie, sowie Grippe; (4) Immunerkrankungen und -leiden, inklusive Autoimmundiabetes, systemischer Lupus erythematosus, GvH-Reaktion, Abstoßung von Fremdtransplantaten, multiple Sklerose, Schuppenflechte und allergische Rhinitis, sowie (5) weitere Erkrankungen und Leiden, darunter Knochenresorptionserkrankungen, Reperfusionsschädigung, sekundäre Kachexie aufgrund Infektion oder Malignität, sekundäre Kachexie aufgrund AIDS, Infektion mit Human Immune Defciency Virus (HIV), oder AIDS-related-Complex (ARC), Keloidbildung, Narbengewebsbildung, Typ 1 Diabetes mellitus sowie Leukämie.

9. Verwendung nach Anspruch 8 einer Verbindung nach Anspruch 1 zur Herstellung eines Arzneimittels zur Behandlung von Myokarderkrankungen.

10. Verwendung nach Anspruch 9 einer Verbindung nach Anspruch 1 zur Herstellung eines Arzneimittels zur Behandlung von Myokarderkrankungen, wobei diese Myokarderkrankungen entzündliche und immunologische Eigenschaften aufweisen.

11. Verwendung nach Anspruch 10 einer Verbindung nach Anspruch 1 zur Herstellung eines Arzneimittels zur Behandlung von koronarer Herzerkrankung, reversibler oder irreversibler Myokardischämie/Reperfusionsschädigung, akutem oder chronischem Herzversagen und Restenose, darunter In-Stent-Restenose und Stent-in-Stent-Restenose.

## Claims

1. Compounds selected from the group
ethyl 5-isopropyl-4-oxo-7-p-tolyl-4,7-dihydro-3*H*-pyrrolo[2,3-*d*]-pyrimidine-6-carboxylate,
ethyl 5-methyl-4-oxo-7-(3-chlorophenyl)-4,7-dihydro-3H-pyrrolo-[2,3-*d*]pyrimidine-6-carboxylate,
ethyl 5-methyl-4-oxo-7-(2-chlorophenyl)-4,7-dihydro-3H-pyrrolo-[2,3-*d*]pyrimidine-6-carboxylate,
ethyl 5-methyl-4-oxo-7-(2-fluorophenyl)-4,7-dihydro-3H-pyrrolo-[2,3-*d*]pyrimidine-6-carboxylate,
ethyl 5-propyl-4-oxo-7-(2-chlorophenyl)-4,7-dihydro-3H-pyrrolo-[2,3-*d*]pyrimidine-6-carboxylate,
ethyl 5-methyl-4-oxo-7-(4-chlorophenyl)-4,7-dihydro-3H-pyrrolo-[2,3-*d*]pyrimidine-6-carboxylate,
methyl 5-methyl-4-oxo-7-(2-chlorophenyl)-4,7-dihydro-3H-pyrrolo-[2,3-*d*]pyrimidine-6-carboxylate,
methyl 5-methyl-4-oxo-7-phenyl-4,7-dihydro-3*H*-pyrrolo[2,3-*d*]-pyrimidine-6-carboxylate,
methyl 5-methyl-4-oxo-7-(2-thienyl)-4,7-dihydro-3*H*-pyrrolo[2,3-*d*]-pyrimidine-6-carboxylate,
and pharmaceutically usable salts, solvates and stereoisomers thereof, including mixtures thereof in all ratios.

2. Compounds according to Claim 1 as phosphodiesterase VII inhibitors.

3. Compounds according to Claim 1 and physiologically acceptable salts and solvates thereof for the treatment or prophylaxis of diseases which can be combated or influenced by compounds having PDE VII-inhibitory activity.

4. Medicaments comprising at least one compound according to Claim 1 and/or pharmaceutically usable salts, solvates and stereoisomers thereof, including mixtures thereof in all ratios, and optionally excipients and/or adjuvants.

5. Use of compounds according to Claim 1 and/or physiologically acceptable salts or solvates thereof for the preparation of a medicament for the treatment of a patient suffering from a disease or disorder mediated by the PDE VII isozyme in its role in the regulation of the activation and degranulation of human eosinophils.

6. Use according to Claim 5 of compounds according to Claim 1 and/or physiologically acceptable salts or solvates thereof for the preparation of a medicament for combating allergic diseases, asthma, chronic bronchitis, atopic dermatitis, psoriasis and other skin diseases, inflammatory diseases, autoimmune diseases, such as, for example, rheumatoid arthritis, multiple sclerosis, Crohn's disease, diabetes mellitus or ulcerative colitis, osteoporosis, transplant rejection reactions, cachexia, tumour growth or tumour metastases, sepsis, memory disorders, atherosclerosis and AIDS.

7. Use according to Claim 5 or 6 of a compound according to Claim 1 for the preparation of a medicament for the treatment or prevention of one or more of the diseases, pathological disorders and conditions from the following group:
asthma of whatever type, etiology or pathogenesis, or asthma from the group atopic asthma, non-atopic asthma, allergic asthma, atopic IgE-mediated asthma, bronchial asthma, essential asthma, true asthma, intrinsic asthma caused by pathophysiological disturbances, extrinsic asthma caused by environmental factors, essential asthma of unknown or unapparent cause, non-atopic asthma, bronchitic asthma, emphysematous asthma, exercise-induced asthma, occupational asthma, infective asthma caused by bacterial, fungal, protozoic or viral infection, non-allergic asthma, incipient asthma, wheezy infant syndrome;
chronic or acute bronchoconstriction, chronic bronchitis, small airway obstruction and emphysema;
obstructive or inflammatory airway diseases of whatever type, etiology or pathogenesis, or an obstructive or inflammatory airway disease from the group asthma, pneumoconiosis, chronic eosinophilic pneumonia, chronic obstructive pulmonary disease (COPD), COPD including chronic bronchitis, pulmonary emphysema or dyspnoea associated therewith, COPD that is **characterised by** irreversible, progressive airway obstruction, adult respiratory distress syndrome (ARDS), and exacerbation of airway hypersensitivity consequent to other medicament therapy;
pneumoconiosis of whatever type, etiology or pathogenesis, or pneumoconiosis from the group aluminosis or bauxite workers' disease, anthracosis or miners' asthma, asbestosis or steam-fitters' asthma, chalicosis or flint disease, ptilosis caused by inhaling the dust from ostrich feathers, siderosis caused by the inhalation of iron particles, silicosis or grinders' disease, byssinosis or cotton-dust pneumoconiosis and talc pneumoconiosis;
bronchitis of whatever type, etiology or pathogenesis, or bronchitis from the group acute bronchitis, acute laryngotracheal bronchitis, arachidic bronchitis, catarrhal bronchitis,
croupous bronchitis, dry bronchitis, infectious asthmatic bronchitis, productive bronchitis, staphylococcal or streptococcal bronchitis and vesicular bronchitis;
bronchiectasis of whatever type, etiology or pathogenesis, or bronchiectasis from the group cylindric bronchiectasis, sacculated bronchiectasis, fusiform bronchiectasis, capillary bronchiectasis, cystic bronchiectasis, dry bronchiectasis and follicular bronchiectasis; seasonal allergic rhinitis, perennial allergic rhinitis, or sinusitis of whatever type, etiology or pathogenesis, or sinusitis from the group purulent or nonpurulent sinusitis, acute or chronic sinusitis, and ethmoid, frontal, maxillary or sphenoid sinusitis;
rheumatoid arthritis of whatever type, etiology or pathogenesis, or rheumatoid arthritis from the group acute arthritis, acute gouty arthritis, chronic inflammatory arthritis, degenerative arthritis, infectious arthritis, Lyme arthritis, proliferative arthritis, psoriatic arthritis and vertebral arthritis;
gout, and fever and pain associated with inflammation;
an eosinophil-related pathological disorder of whatever type, etiology or pathogenesis, or an eosinophil-related pathological disorder from the group eosinophilia, pulmonary infiltration eosinophilia, Löffler's syndrome, chronic eosinophilic pneumonia, tropical pulmonary eosinophilia, bronchopneumonic aspergillosis, aspergilloma, granulomas containing eosinophils, allergic granulomatous angiitis or Churg-Strauss syndrome, polyarteritis nodosa (PAN) and systemic necrotising vasculitis;
atopic dermatitis, allergic dermatitis, or allergic or atopic eczema; urticaria of whatever type, etiology or pathogenesis, or urticaria from the group immune-mediated urticaria, complement-mediated urticaria, urticariogenic material-induced urticaria, physical stimulus-induced urticaria, stress-induced urticaria, idiopathic urticaria, acute urticaria, chronic urticaria, angiooedema, cholinergic urticaria, cold urticaria in the autosomal dominant form or in the acquired form, contact urticaria, giant urticaria and papular urticaria;
conjunctivitis of whatever type, etiology or pathogenesis, or conjunctivitis from the group actinic conjunctivitis, acute catarrhal conjunctivitis, acute contagious conjunctivitis, allergic conjunctivitis, atopic conjunctivitis, chronic catarrhal conjunctivitis, purulent conjunctivitis and vernal conjunctivitis;
uveitis of whatever type, etiology or pathogenesis, or uveitis from the group inflammation of all or part of the uvea, anterior uveitis, iritis, cyclitis, iridocyclitis, granulomatous uveitis, nongranulomatous uveitis, phacoantigenic uveitis, posterior uveitis, choroiditis and chorioretinitis;
psoriasis;
multiple sclerosis of whatever type, etiology or pathogenesis, or multiple sclerosis from the group primary progressive multiple sclerosis and relapsing/remitting multiple sclerosis; autoimmune/inflammatory diseases of whatever type, etiology or pathogenesis, or an autoimmune/inflammatory disease from the group autoimmune haematological disorders, haemolytic anaemia, aplastic anaemia, pure red cell anaemia, idiopathic thrombocytopenic purpura, systemic lupus erythematosus, polychondritis, scleroderma, Wegener's granulomatosis, photodermatosis, chronic active hepatitis, myasthenia gravis, Stevens-Johnson syndrome, idiopathic sprue, autoimmune inflammatory bowel diseases, ulcerative colitis, Crohn's disease, endocrine ophthamopathy, Grave's disease, sarcoidosis, alveolitis, chronic hypersensitivity pneumonitis, primary biliary cirrhosis, insulin deficiency diabetes or type 1 diabetes mellitus, anterior uveitis, granulomatous or posterior uveitis, keratoconjunctivitis sicca, epidemic keratoconjunctivitis, (diffuse) interstitial pulmonary fibrosis, cystic fibrosis, psoriatic arthritis, glomerulonephritis with and without nephrosis syndrome, acute glomerulonephritis, idiopathic nephrosis syndrome, minimal change nephropathy, inflammatory/ hyperproliferative skin diseases, psoriasis, atopic dermatitis, contact dermatitis, allergic contact dermatitis, benign familial pemphigus, pemphigus erythematosus, pemphigus foliaceus and pemphigus vulgaris;
prevention of foreign transplant rejection following organ transplantation;
inflammatory bowel disease (IBD) of whatever type, etiology or pathogenesis, or inflammatory bowel disease from the group ulcerative colitis (UC), collagenous colitis, colitis polyposa, transmural colitis and Crohn's disease (CD);
septic shock of whatever type, etiology or pathogenesis, or septic shock from the group renal failure, acute renal failure, cachexia, malarial cachexia, hypophysial cachexia, uremic cachexia, cardiac cachexia, cachexia suprarenalis or Addison's disease, cancerous cachexia, and cachexia as a consequence of infection by the human immunodeficiency virus (HIV); liver damage;
pulmonary hypertension and hypoxia-induced pulmonary hypertension;
bone loss diseases, primary osteoporosis and secondary osteoporosis;
pathological disorders of the central nervous system of whatever type, etiology or pathogenesis, or a pathological disorder of the central nervous system from the group depression, Parkinson's disease, learning and memory disorders, tardive dyskinesia, drug dependence, arteriosclerotic dementia, and dementias that accompany Huntington's chorea, Wison's disease, paralysis agitans and thalamic atrophies;
infections, especially viral infections, where these viruses
increase the production of TNF-α in their host or where these viruses are sensitive to up-regulation of TNF-α in their host so that their replication or other vital activities are adversely affected, including viruses from the group HIV-1, HIV-2 and HIV-3, cytomegalovirus, CMV, influenza, adenoviruses and Herpes viruses, including Herpes zoster and Herpes simplex;
yeast and fungal infections, where these yeasts and fungi are sensitive to up-regulation by TNF-α or elicit TNF-α production in their host, for example fungal meningitis, particularly when administered in conjunction with other medicaments of choice for the treatment of systemic yeast and fungal infections, including, but not limited to, polymycins, for example polymycin B, imidazoles, for example clotrimazole, econazole, miconazole and ketoconazole, triazoles, for example fluconazole and itranazole, and amphotericins, for example amphotericin B and liposomal amphotericin B;
ischaemia-reperfusion damage, autoimmune diabetes, retinal autoimmunity, chronic lymphocytic leukaemia, HIV infections, lupus erythematosus, kidney and ureter diseases, pathological urogenital and gastrointestinal disorders, and prostate diseases.

8. Use according to Claim 5, 6 or 7 of a compound according to Claim 1 for the preparation of a medicament for the treatment of (1) inflammatory diseases and conditions, including joint inflammation, rheumatoid arthritis, rheumatoid spondylitis, osteoarthritis, inflammatory bowel disease, ulcerative colitis, chronic glomerulonephritis, dermatitis and Crohn's disease, (2) respiratory tract diseases and conditions, including asthma, adult respiratory distress syndrome, chronic pulmonitis, bronchitis, chronic obstructive airway disease and silicosis, (3) infectious diseases and conditions, including sepsis, septic shock, endotoxic shock, Gram-negative sepsis, toxic shock syndrome, fever and myalgia due to bacterial, viral or fungal infections, and influenza, (4) immune diseases and conditions, including autoimmune diabetes, systemic lupus erythematosus, GvH reaction, rejection of foreign transplants, multiple sclerosis, psoriasis and allergic rhinitis, and (5) other diseases and conditions, including bone absorption diseases, reperfusion damage, cachexia secondary to infection or malignancy, cachexia secondary to AIDS, human immunodeficiency virus (HIV) infection, or AIDS-related complex (ARC), keloid formation, scar tissue formation, type 1 diabetes mellitus and leukaemia.

9. Use according to Claim 8 of a compound according to Claim 1 for the preparation of a medicament for the treatment of myocardial diseases.

10. Use according to Claim 9 of a compound according to Claim 1 for the preparation of a medicament for the treatment of myocardial diseases, where these myocardial diseases have inflammatory and immunological properties.

11. Use according to Claim 10 of a compound according to Claim 1 for the preparation of a medicament for the treatment of coronary heart disease, reversible or irreversible myocardial ischaemia/reperfusion damage, acute or chronic heart failure and restenosis, including in-stent restenosis and stent-in-stent restenosis.

## Revendications

1. Composés sélectionnés parmi le groupe
le 5-isopropyl-4-oxo-7-p-tolyl-4,7-dihydro-3*H*-pyrrolo[2,3-*d*]pyrimidine-6-carboxylate d'éthyle,
le 5-méthyl-4-oxo-7-(3-chlorophényl)-4,7-dihydro-3*H*-pyrrolo[2,3-*d*]-pyrimidine-6-carboxylate d'éthyle,
le 5-méthyl-4-oxo-7-(2-chlorophényl)-4,7-dihydro-3*H*-pyrrolo[2,3-*d*]-pyrimidine-6-carboxylate d'éthyle,
le 5-méthyl-4-oxo-7-(2-fluorophényl)-4,7-dihydro-3*H*-pyrrolo[2,3-*d*]-pyrimidine-6-carboxylate d'éthyle,
le 5-propyl-4-oxo-7-(2-chlorophényl)-4,7-dihydro-3*H*-pyrrolo[2,3-*d*]-pyrimidine-6-carboxylate d'éthyle,
le 5-méthyl-4-oxo-7-(4-chlorophényl)-4,7-dihydro-3*H*-pyrrolo[2,3-*d*]-pyrimidine-6-carboxylate d'éthyle,
le 5-méthyl-4-oxo-7-(2-chlorophényl)-4,7-dihydro-3*H*-pyrrolo[2,3-*d*]-pyrimidine-6-carboxylate de méthyle,
le 5-méthyl-4-oxo-7-phényl-4,7-dihydro-3*H*-pyrrolo[2,3-*d*]pyrimidine-6-carboxylate de méthyle,
le 5-méthyl-4-oxo-7-(2-thiényl)-4,7-dihydro-3*H*-pyrrolo[2,3-*d*]pyrimidine-6-carboxylate de méthyle,
et leurs sels, solvats et stéréoisomères pharmaceutiquement utilisables, y compris leurs mélanges en toutes proportions.

2. Composés selon la revendication 1, comme inhibiteurs de la phosphodiestérase VII.

3. Composés selon la revendication 1 et leurs sels et solvats physiologiquement acceptables, pour le traitement ou la prophylaxie de maladies pouvant être combattues ou influencées par des composés ayant une activité inhibitrice de la PDE VII.

4. Médicaments comprenant au moins un composé selon la revendication 1 et/ou leurs sels, solvates et stéréoisomères pharmaceutiquement utilisables, y compris leurs mélanges afférents selon tous rapports, et en option des excipients et/ou des adjuvants.

5. Utilisation de composés selon la revendication 1 et/ou de leurs sels ou solvates physiologiquement acceptables pour la préparation d'un médicament pour le traitement d'un patient qui souffre d'une maladie ou d'une condition favorisée par l'isozyme PDE VII dans son rôle de régulation de l'activation et de la dégranulation des éosinophiles humains.

6. Utilisation selon la revendication 5 de composés selon la revendication 1 et/ou de leurs sels ou solvates physiologiquement acceptables pour la préparation d'un médicament pour combattre des maladies allergiques, l'asthme, la bronchite chronique, la dermatite atopique, le psoriasis et d'autres maladie de la peau, des maladies inflammatoires, des troubles auto-immunes tels que par exemple l'arthrite rhumatoïde, la sclérose en plaques, la maladie de Crohn, le diabète sucré ou la recto-colite hémorragique, l'ostéoporose, les réactions de rejet de transplants, le marasme, la croissance tumorale ou les métastases tumorales, la sepsie, les troubles de la mémoire, l'athérosclérose et le SIDA.

7. Utilisation selon la revendication 5 ou 6 d'un composé selon la revendication 1 pour la préparation d'un médicament pour le traitement ou la prévention d'une ou de plusieurs maladies, d'un ou de plusieurs troubles et conditions pris parmi le groupe :
l'asthme de tout type, toute étiologie ou toute pathogenèse ; ou l'asthme qui est un élément choisi parmi le groupe comprenant l'asthme atopique ; l'asthme non atopique ; l'asthme allergique ; l'asthme favorisé par IgE bronchial atopique ; l'asthme bronchial ; l'asthme essentiel ; l'asthme vrai ; l'asthme intrinsèque généré par des perturbations pathophysiologiques ; l'asthme extrinsèque généré par des facteurs environnementaux ; l'asthme essentiel de cause inconnue ou inapparente ; l'asthme non atopique ; l'asthme bronchitique ; l'asthme emphysémateux ; l'asthme induit par l'exercice ; l'asthme professionnel ; l'asthme infectieux-allergique généré par une infection bactérienne, fongique, protozoale ou virale ; l'asthme non allergique ; l'asthme incipiens ; le syndrome du petit enfant asthmatique ; la bronchoconstriction chronique ou aiguë ; la bronchite chronique ; une obstruction légère des voies aériennes ; et l'emphysème ;
les maladies obstructrices ou inflammatoires des voies aériennes de tout type, toute étiologie ou toute pathogénèse ; ou une maladie obstructrice ou inflammatoire des voies aériennes qui est un élément choisi parmi le groupe comprenant l'asthme ; la pneumoconiose ; la pneumonie éosinophilique chronique ; la maladie pulmonaire obstructrice chronique (COPD) ; la COPD qui inclut la bronchite chronique, l'emphysème pulmonaire ou la dyspnée associée ; la COPD qui est **caractérisée par** une obstruction progressive irréversible des voies aériennes ; le syndrome de détresse respiratoire de l'adulte (ARDS) et une exacerbation d'une hyperactivité des voies aériennes en tant que résultat d'une thérapie avec d'autres médicaments ;
la pneumoconiose de tout type, toute étiologie ou toute pathogenèse ; ou la pneumoconiose qui est un élément choisi parmi le groupe comprenant l'aluminose ou la maladie des travailleurs de la bauxite ; l'anthracose ou l'asthme des mineurs ; l'amiantose ou l'asthme des filtres à vapeur ; la silicose ou la maladie de flint ; la trichoptilose générée par l'inhalation de poussières issues de plumes d'autruches ; la sidérose générée par l'inhalation de particules de fer ; la silicose ou la maladie des meuleurs ; la byssinose ou l'asthme de la poussière de coton ; et la pneumoconiose du talc ;
la bronchite de tout type, toute étiologie ou toute pathogenèse ; ou la bronchite qui est un élément choisi parmi le groupe comprenant la bronchite aiguë ; la bronchite laryngotrachéale aiguë ; la bronchite arachidique ; la bronchique catarrhale ; la bronchite croupus ; la bronchite sèche ; la bronchite asthmatique infectieuse ; la bronchite productive ; la bronchite à staphylocoque ou streptocoque ; et la bronchopneumonie ;
la bronchectasie de tout type, toute étiologie ou toute pathogenèse ; ou la bronchectasie qui est un élément choisi parmi le groupe comprenant la bronchectasie cylindrique ; la bronchectasie sacculaire ; la bronchectasie fusocellulaire ; la bronchectasie capillaire ; la bronchectasie cystique ; la bronchectasie sèche ; et la bronchectasie folliculaire ;
la rhinite allergique saisonnière ; ou la rhinite allergique apériodique ; ou la sinusite de tout type, toute étiologie ou toute pathogénèse ; ou la sinusite qui est un élément choisi parmi le groupe comprenant la sinusite purulente ou non purulente ; la sinusite aiguë ou chronique ; et la sinusite ethmoïde, frontale, maxillaire ou sphénoïde ;
l'arthrite rhumatoïde de tout type, toute étiologie ou toute pathogénèse ; ou l'arthrite rhumatoïde qui est un élément choisi parmi le groupe comprenant l'arthrite aiguë ; l'arthrite goutteuse aiguë ; l'arthrite inflammatoire chronique ; l'arthrite dégénérative ; l'arthrite infectieuse ; l'arthrite de Lyme ; l'arthrite proliférative ; l'arthrite psoriatique ; et l'arthrite vertébrale ;
la goutte ainsi que la fièvre et la douleur associées à une inflammation ;
un trouble pathologique rapporté à l'éosinophile de tout type, toute étiologie ou toute pathogénèse ; ou un trouble rapporté à l'éosinophile qui est un élément choisi parmi le groupe comprenant l'éosinophilie ; l'éosinophilie par infiltration pulmonaire ; le syndrome de Loffier ; la pneumonie éosinophilique chronique ; l'éosinophilie pulmonaire tropicale ; l'aspergillose bronchopneumonique ; l'aspergillome ; les granulomes éosinophiliques ; l'angéite granulomateuse allergique ou le syndrome de Churg-Strauss ; la polyartérite noueuse (PAN) ; et l'angéite nécrosante systémique ;
la dermatite atopique ; ou la dermatite allergique ; ou l'eczéma allergique ou atopique ;
l'urticaire de tout type, toute étiologie ou toute pathogénèse ; ou l'urticaire qui est un élément choisi parmi le groupe comprenant l'urticaire immunofavorisée ; l'urticaire favorisée par complément ; l'urticaire induite par des matériaux urticariogéniques ; l'urticaire induite par agent physique ; l'urticaire induite par stress ; l'urticaire idiopathique ; l'urticaire aiguë ; l'urticaire chronique ; l'oedème angioneurotique ; l'urticaire cholinergique ; l'urticaire au froid sous la forme dominante autosomique ou sous la forme acquise ; l'urticaire par contact ; l'urticaire géante ; et l'urticaire papuleuse ;
la conjonctivite de tout type, toute étiologie ou toute pathogénèse ; ou la conjonctivite qui est un élément choisi parmi le groupe comprenant la conjonctivite actinique, la conjonctivite catarrhale aiguë ; la conjonctivite contagieuse aiguë ; la conjonctivite allergique ; la conjonctivite atopique ; la conjonctivite catarrhale chronique ; la conjonctivite purulente ; et la conjonctivite printanière ;
l'uvéite de tout type, toute étiologie ou toute pathogénèse ; ou l'uvéite qui est un élément choisi parmi le groupe comprenant l'inflammation de la totalité ou d'une partie de l'uvée ; l'uvéite antérieure ; l'iritis ; la cyclite ; l'iridocyclite ; l'uvéite granulomateuse ; l'uvéite non granulomateuse ; l'uvéite phacoantigénique ; l'uvéite postérieure ; la choroïdite ; et la choriorétinite ;
le psoriasis ;
la sclérose en plaques de tout type, toute étiologie ou toute pathogénèse ; ou la sclérose en plaques qui est un élément choisi parmi le groupe comprenant la sclérose en plaques progressive primitive ; et la sclérose en plaques cyclique ;
les maladies auto-immunes/inflammatoires de tout type, toute étiologie ou toute pathogénèse ; ou une maladie auto-immunelinflammatoire qui est un élément choisi parmi le groupe comprenant des troubles hématologiques auto-immunes ; l'anémie hémolytique ; l'anémie aplastique ; l'anémie érythroblastopénique ; la purpura thrombopénique idiopathique ; le Lupus Erythematosus systémique; la polychondrite ; la sclérodermie ; la granulomatose de Wegner ; la dermatomyosite ; l'hépatite active chronique ; la myasthénie gravis ; le syndrome de Stevens-Johnson ; la sprue idiopathique ; les maladies inflammatoires auto-immunes de l'intestin ; la recto-colite hémorragique ; la maladie de Crohn ; l'ophthalmopathie endocrine ; la maladie de Grave ; la sarcoïdose ; l'alvéolite ; la pneumonite à hypersensibilité chronique ; la cirrhose biliaire primitive ; le diabète juvénile ou le diabète sucré de type 1; l'uvéite antérieure ; l'uvéite granulomateuse ou postérieure ; la kératoconjonctivite sèche ; la kératoconjonctivite épidémique ; la fibrose pulmonaire interstitielle diffuse ou la fibrose des poumons interstitielle diffuse ; la fibrose pulmonaire idiopathique ; la fibrose cystique ; l'arthrite psoriatique ; la glomérulonéphrite avec et sans syndrome néphrotique ; la glomérulonéphrite aiguë ; le syndrome néphrotique idiopathique ; la néphropathie à variation minimum ;
les maladies de la peau inflammatoires/hyperprolifératives ; le psoriasis ; la dermatite atopique ; la dermatite par contact ; la dermatite par contact allergique ; le pemphigus familial bénin ; le Pemphigus Erythematosus ; le Pemphigus Foliaceus ; et le Pemphigus Vulgaris ;
la prévention du rejet de transplant étranger suite à une transplantation d'organe ;
une affection abdominale inflammatoire (IBD) de tout type, toute étiologie ou toute pathogénèse ; ou une affection abdominale inflammatoire qui est un élément choisi parmi le groupe comprenant la recto-colite hémorragique (UC) ; la colite collagène ; la colite polypose ; la colite transmurale ; et la maladie de Crohn (CD) ;
un choc septique de tout type, toute étiologie ou toute pathogénèse ; ou un choc septique qui est un élément choisi parmi le groupe comprenant une insuffisance rénale ; l'insuffisance rénale aiguë ; le marasme ; le marasme paludéen ; le marasme hypophysique ; le marasme urémique ; le marasme cardiaque ; le marasme suprarénal ou la maladie d'Addison ; le marasme cancéreux ; et le marasme en tant que conséquence d'une infection par le virus immunodéficitaire humain (VIH) ;
un endommagement du foie ;
une hypertension pulmonaire ; et une hypertension pulmonaire induite par hypoxie ;
les maladies de perte osseuse ; l'ostéoporose primitive ; et l'ostéoporose secondaire ;
les troubles pathologiques du système nerveux central de tout type, toute étiologie ou toute pathogénèse ; ou un trouble pathologique du système nerveux central qui est un élément choisi parmi le groupe comprenant une dépression ; la maladie de Parkinson ; une altération de l'apprentissage et de la mémoire ; la dyskinésie tardive ; la dépendance aux médicaments ; la démence artériosclérotique ; et les démences qui accompagnent la chorée de Huntington, la maladie de Wilson, la paralysie agitante et les atrophies thalamiques ; une infection, tout particulièrement une infection due à des virus
selon laquelle ces virus augmentent la production de TNF-α dans leurs hôtes ou selon laquelle ces virus sont sensibles à une régulation à la hausse de TNF-α dans leurs hôtes de telle sorte que leur réplication ou leurs autres activités vitales subissent un impact défavorable, incluant un virus qui est un élément choisi parmi le groupe comprenant VIH-1, VIH-2 et VIH-3 ; le cytomégalovirus, soit CMV ; la grippe ; les adénovirus ; et les virus de l'herpès, incluant le Herpes Zoster ou zona d'herpès et le Herpes Simplex ;
les infections dues aux levures et aux champignons où lesdites levures et lesdits champignons sont sensibles à une régulation à la
hausse par TNF-α ou élisent une production de TNF-α au niveau de leurs hôtes, par exemple une méningite fongique ; plus particulièrement lors d'une administration en conjonction avec d'autres médicaments de choix pour le traitement des infections par levures et
champignons systémiques incluant, sans qu'on soit limité à cela, les polymycines, par exemple Polymycin B ; les imidazoles, par exemple clotrimazole, éconazole, miconazole et cétoconazole ; les triazoles, par exemple fluconazole et itranazole ; et les amphotéricines, par exemple Amphotericin B et Amphotericin B liposomal ;
la blessure de l'ischémie-reperfusion ; les diabètes auto-immunes ; l'auto-immunité rétinienne ; la leucémie lymphocytique chronique ; les infections VIH ; le Lupus Erythematosus ; une maladie du foie et de l'uretère ; les troubles pathologiques urogénitaux et gastro-intestinaux ; et les maladies de la prostate.

8. Utilisation selon la revendication 5, 6 ou 7 d'un composé selon la revendication 1 pour la préparation d'un médicament pour le traitement (1) des maladies inflammatoires et des conditions inflammatoires comprenant une inflammation articulaire, l'arthrite rhumatoïde, la spondylite rhumatoïde, l'ostéoarthrite, l'affection abdominale inflammatoire, la recto-colite hémorragique, la glomérulonéphrite chronique ; la dermatite et la maladie de Crohn ; (2) des maladies respiratoires et des conditions respiratoires comprenant : l'asthme, le syndrome de détresse respiratoire de l'adulte, la maladie inflammatoire pulmonaire chronique, la bronchite, la maladie obstructrice chronique des voies aériennes et la silicose ; (3) des maladies infectieuses et des conditions infectieuses comprenant : la sepsie, le choc septique, le choc endotoxique, le gram-négatif, la sepsie, le syndrome de choc toxique, la fièvre et la myalgie dues à une infection bactérienne, virale ou fongique et la grippe ; (4) des maladies et des conditions immunes comprenant : des diabètes auto-immunes, le lupus erythematosus systémique ; une réaction GvH ; des rejets de transplants étrangers, la sclérose en plaques, le psoriasis et la rhinite allergique ; et (5) d'autres maladies et d'autres conditions comprenant des maladies de la résorption osseuse ; une blessure de reperfusion ; un marasme secondaire résultant d'une infection ou d'une tumeur maligne ; un marasme secondaire résultant du SIDA, une infection du virus immunodéficitaire humain (VIH) ou un complexe rapporté au SIDA (ARC) ; une formation de chéloïde ; une formation de tissu cicatriciel ; un diabète sucré de type 1 ; et la leucémie.

9. Utilisation selon la revendication 8 d'un composé selon la revendication 1 pour la préparation d'un médicament pour le traitement des maladies du myocarde.

10. Utilisation selon la revendication 9 d'un composé selon la revendication 1 pour la préparation d'un médicament pour le traitement de maladies du myocarde, où ces maladies du myocarde présentent des propriétés inflammatoires et immunologiques.

11. Utilisation selon la revendication 10 d'un composé selon la revendication 1 pour la préparation d'un médicament pour le traitement d'une maladie des artères coronaires, un dommage par ischémie/perfusion myocardiale réversible ou irréversible, une défaillance cardiaque aiguë ou chronique et une resténose, incluant une resténose intra-stent et une resténose stent-dans-stent.
